# EUROPEAN PATENT APPLICATION

(11) **EP 2 364 698 A2**
(43) Date of publication of application: **14.09.2011**
(21) Application number: 09800592.9
(22) Date of filing: 24.07.2009
(51) Int. Cl.: A61K 31/135

(54) **COMPOSITION FOR CONTROL OF AGING AND / OR EXTENSION OF LIFE, CONTAINING DAPSONE AS ACTIVE INGREDIENT**

(30) Priority: 25.07.2008 KR 20080072762
(71) Applicant: SNU R&DB Foundation, Gwanak-gu Seoul 151-015 (KR)
(72) Inventor: PARK, Sang Chul, Seongnam-si Gyeonggi-do 463-470 (KR); LEE, Jun Ho, Seoul 135-554 (KR); CHO, Sung Chun, Incheon 407-801 (KR); PARK, Moon Cheol, Seoul 158-076 (KR); CHO, Yun Je, Pohang-si Gyeongsangbuk-do 790-751 (KR)
(74) Representative: Catherine, Alain
(86) International application number: PCT/KR2009/004148
(87) International publication number: WO 2010/011111

(57) **Abstract**

The present invention relates to a composition for controlling aging and/or extending lifespan. More specifically, the present invention relates to a composition containing dapsone as an effective component for controlling aging and/or extending lifespan, in which when applying the composition according to the present invention to an object, the effect of controlling aging or extending lifespan is excellent. Specifically, the treatment of dapsone (DDS) increases the lifespan of *C. elegans* not depending upon daf-16 and delays aging process, and reduces the activity of cytoplasmic dual oxidase 1 (Duox1) and mitochondria complex V protein. In addition, dapsone can maintain the low oxygen uptake rate in *C. elegans* thereby reducing ROS formation. In addition, ROS production by paraquat that is an inducing material of oxidative stress is reduced by treating dapsone and the sensitivity of paraquat is reduced. Moreover, it can be known that pyruvate kinase is a target material of dapsone through the structural prediction, molecular and biochemical analysis. In addition, there is an effect that the recovery of mitochondria function that is reduced and the decrease of NOX expression in a cell are obtained by dapsone in the human diploid fibroblast. From the comprehensive results, it can be known that dapsone has an excellent effect for extending lifespan in a cell level and object level.

## Description

### [Technical Field]

The present invention relates to a composition for controlling aging and/or extending lifespan. More specifically, the present invention relates to a composition including dapsone as an active ingredient for controlling aging and/or extending lifespan.

### [Background Art]

Aging is broadly divided into a damage accumulation theory of aging and a genetic program theory of aging. Specifically, the damage accumulation theory of aging is that aging is caused by a continuous accumulation of cell damages, a hypoergy due to the denaturation of the protein in a cell and membrane lipid, directly and indirectly DNA damage, a malfunction of mitochondria, and the like by a harmful product produced from a metabolic process of an object, such as various environmental harmful elements and an oxygen radical, in which the object is continuously exposed to the harmful products during the lifespan of object. Meanwhile, the genetic program theory of aging is that aging is caused by the development and growth of object according to the genetic program by being inherent kinds of biological clock in genes of all objects, like a telomere hypothesis of cell aging.

Meanwhile, dapsone (4,4'-diaminodiphenylsulfone; hereinafter called as dapsone or DDS) is a material synthesized before a century, is well known as a medicine for Hansen's disease, and is used for other many skin diseases as an most common drug [1, 40]. In addition, even if it is not clear that DDS functions as an oxidant or antioxidant, some researches suggest that DDS functions as an oxidant and the use of DDS should be restricted due to an induction of hemolytic anemia [2, 3]. Some researches suggest that DDS can function as an antioxidant [5, 6]. However, the dapsone is not known to be effective in controlling aging, preventing aging or extending lifespan.

It is required that the molecular mechanism should be identified from the researches about aging at the level of cell, i.e., cellular senescence in order to understand aging of object. Many researches about the cellular senescence are mainly achieved by using a human fibroblast [41], in which the aging process of object is well reported in human.

### [Disclosure]

### [Technical Problem]

The inventors finished the present invention by discovering the composition including dapsone (DDS) that has an excellent effect for extending lifespan and has an excellent effect for suppressing cytotoxicity in *C*. *elegans* that is induced by paraquat in a human fibroblast. The composition including dapsone according to the present invention has effects for reducing the function of mitochondria in *C*. *elegans* and for reducing the expression of ROS-generating proteins of cytoplasm, and also it has excellent effects for recovering the reduced function of mitochondria and for reducing the expressing of NOX in a human fibroblast.

Thus, an object of the present invention is to provide the composition including dapsone as an effective component for controlling aging and/or extending lifespan.

Another object of the present invention is to provide a method for extending lifespan of object, in which the method includes applying an effective amount of dapsone to an object.

Another object of the present invention is to provide a method for controlling aging of cell, in which the method includes applying the composition to aging cell.

Other objects and advantages of the present invention will be described in more detail with reference to the following description along with the accompanying claims and drawings.

In addition, many cited references and patent documents would be referenced and the quotations would be marked over all the description. The disclosed contents in the cited references and the patents would be inserted as a reference in the description as a whole so that the content of the present invention and the level of the technical field, to which the present invention belongs, would be described in more clearly.

### [Technical Solution]

The present invention is characterized in that the composition for controlling aging and/or extending lifespan includes dapsone in order to alleviate or suppress aging of object in matters of aging, in which objects all live their lives-experiencing aging. Thus, an object of the present invention is to provide the composition including dapsone according to the present invention for controlling aging of object and aging of cell.

According to an embodiment of the present invention, the present invention is to provide the composition including dapsone as an effective component for controlling aging and/or extending lifespan.

The inventors exerted all possible effects for finding whether or not the composition including dapsone (DDS) that is a drug for widely using the treatments of Hansen's disease, various skin diseases, and malaria has an effects for extending lifespan of object. As a result, the inventors found out that it has an excellent effect for extending lifespan of object.

The term, "senescene" used in the description has the same meaning as aging. Thus, "Controlling senescense" means everything for controlling aging, such as suppressing aging, preventing aging, alleviating aging, and the like. As more specific examples, it means that the senescense of cell is temporarily suppressed by using the composition according to the present invention, or recovering the biological function of aging cell appears a similar biological phenomenon as a young cell. For example, for the aging cell that is treated with the composition according to the present invention, the reactivity about a growth factor, such as EGF, is recovered, so that the signal transduction is recovered by the growth factor and the cell cycle is normally operated. In addition, the suppression or alleviation of the cellular senescense may directly or indirectly effect to lifespan so that aging of object can ultimately be alleviated.

Various animals including a mammalian as a model animal can be used in order to identify the effect of dapsone on a lifespan of object, but the present invention uses *C*. *elegans*.

*C. elegans* is a nematoda that lives by eating bacteria in a soil, does not have legs, wings, eyes or an arthromere, and senses an ambient temperature, smells, and the like through a sensory organ located in a head or tail. It has a approximately 1 mm as an adult size, is a multicellular organism that has a relatively simple shape, has a transparent body, and is a hermaphrodite, but there is a arrhenotoky *C. elegans*. Since it has been started to use as a experiment model in 70's, it is being used widely due to the usability of handling and it has an advantage such that since the 99's genome project was finished so that genetic map and base sequence of six chromosome map are easy to access. In addition, *C. elegans* can be used to research the study for extending lifespan as a useful model, because it becomes a nematode via an egg-stage after hatching from egg, a four-stage ecdysis, consisted in the larva-stage of L1 to L4, the above period is three-day, and the average lifespan is short to be about 2-3 weeks.

According to a specific embodiment of the present invention, the dapsone effect in a lifespan of object by using *C. elegans* is observed as follows: firstly *E. coli* was cultured in a medium including dapsone, *C. elegans* is grown by using the cultured *E. coli* as a food, and then their lifespan is observed. The dapsone that is an antibiotic makes a growth of *E. coli* to reduce so that it may be affected to the lifespan of *C. elegans*. Thus, in order to prevent the above dietary restriction, PABA (para-aminobenzoic acid) that is competitively suppressed by dapsone is added to the cultural medium so that the growth of *E*. *coli* is maintained in a similar level as a control. At this time, the concentration of PABA is about 10 uM that can affect only to the growth of *E. coli* so that the above concentration is to ensure that PABA could not affect to the lifespan of *C. elegans*.

In addition, according to a specific embodiment of the present invention, the mechanism for extending the lifespan of *C. elegans* can be modulated by an insulin signal, a mitochondria complex, a pyruvate kinase and ROS of cytoplasm. More specifically, the extending of lifespan in *C. elegans* by DDS can be achieved from a decrease in controlling an insulin signal, a decrease in a function of mitochondria (such as, a decrease in a transmembrane potential of mitochondria, a decrease of the content of mitochondria complex, etc, a decrease of the content of ATP in cell, a decrease in an oxygen uptake rate in a cell, and the like), a decrease in an expression of ROS-generating proteins in a cytoplasm (such as, suppression in generating ROS by the decrease of mRNA level in a cytoplasm, the content of ROS-generating protein, and the like), and an increase of the pyruvate content (such as, pyruvate kinase gene mutant - specifically the pyruvate kinase activity in a muscle).

The mitochondria complex in the mechanism for extending lifespan by DDS is preferably a complex V, and the pyruvate kinase gene mutant is preferably pyk-1.

In addition, the inventors made an effect for finding the effect of controlling aging in a level of human cell about the composition including dapsone (DDS), and as a result, the inventors found that the composition including dapsone (DDS) has an excellent effect for suppressing ROS-generating material in a cytoplasm and controlling the function of mitochondria in a cell that are involved in the effect for extending the lifespan of object.

Thus, according a specific embodiment of the present invention, the present invention is performed by using a human fibroblast to identify whether or not dapsone can suppress a cytotoxicity generated by paraquat after increasing an oxidative stress that is one of mechanisms related to the aging by paraquat.

A drug used in the present invention, i.e., paraquat (hereinafter, called as paraquat or PQ) is a kind of herbicide, and causes the serious damage of cell through the oxidation process. PQ is widely used for inducing an oxidative stress in a cell by generating a superoxide anion in a metabolism process depending on NADPH. A protein kinase C (PKC) is a necessary element for BADPH oxidass (NOX) that is generated by starting by a ligand in order to generate the superoxide anion [7]. Recently, PQ is reported to generate ROS through the activity of NOX depending on PKC-delta [8]. In addition, a mitochondria is considered to be importantly involved in inducing the cytotoxicity due to the oxidative stress that is induced by PG because the mitochondria is a main source of ROS. Recent research shows that a mitochondria complex I (NADH-ubiquinone oxido-reductase) is an important place of mitochondria in order to generate the superoxide anion by PQ [9]. Another research reported the result such that PQ makes a mitochondria complex V activity to meaningfully decrease [10]. The imperfection in functions of mitochondria organs and the decrease of mitochondria complex by PQ provide the results in inducing the generation of the superoxide anion and the suppression of the electron transport [11]. An antioxidant suppress the generation of ROS, the elimination of ROS and the damage generated by ROS so that it can be important in preventing various diseases of human that are induced by the oxidative stress if it has a effective anti-oxidative function and also stability.

According to a specific embodiment of the present invention, the composition including dapsone according to the present invention can control a cell aging by reducing the expression of NOX due to the suppression of PKC activity that is induced by paraquat, and effectively controlling an abnormal function of mitochondria, i.e., the change of the amount of complex protein of mitochondria, the change of membrane potential, the increase of ROS generation, and the like.

The composition according to the present invention may be made as a dosage form for a cosmetic, a pharmacy, and a healthy food or drink through the general method that is well known in the relevant field.

When the composition according to the present invention is prepared as the composition for a cosmetic, the composition includes the components that are generally used for the cosmetic composition in addition to dapsone as the above effective component, and more specifically includes common adjurvants, such as an antioxidant, a stabilizer, a solubilizer, a vitamin, a pigment and flavoring, and a carrier. The cosmetic composition according to the present invention can be prepared as any types of dosage forms that are generally formed in the art, and for example can be prepared as a dosage form, such as a solution, a suspension, an emulsion, a paste gel, a cream, a lotion, a powder, a soup, a surfactant-containing cleansing, an oil, a powder foundation, an emulsion foundation, a wax foundation a spray, and the like, but is not limited thereto. More specifically, the cosmetic composition according to the present invention can be prepared as a dosage form, such as an astringent, a nutrient tonic, an extra rich cream, a massage cream, an essence, an eye cream, a cleansing cream, a cleansing form, a cleansing water, a pack, a spray or a powder.

When the composition according to the present invention is prepared as a pharmaceutical composition or a healthy food, the composition may include a pharmaceutical acceptable carrier or a carrier that is acceptable for food in addition to dapsone, and more specifically it may include a carbohydrate-based composition (examples: lactose, amylose, dextrose, sucrose, sorbitol, mannitol, starch, cellulose, and the like), an acacia rubber, calcium phosphate, alginate, gelatin, calcium silicate, microcrystalline cellulose, polyvinylpyrrolidone, cellulose, water, syrup, salt solution, alcohol, arabia rubber, vegetable oil (examples: corn oil, cotton seed oil, soybean oil, olive oil, coconut oil), polyethylene glycol, methyl cellulose, methyl hydroxy benzoate, propyl hydroxy benzoate, talc, stearate magnesium, mineral oil, and the like, but is not limited thereto. The composition according to the present invention may further include a lubricant, a wetting agent, a sweeting agent, a flavor, an emulsifier, a suspension, preservatives, and the like in addition to the above components.

The composition according to the present invention may be prepared in a type of unit capacity or in putting within a multicapacity container by formulating using a pharmaceutical acceptable carrier and/or an excipient according to the method that can be easily performed by the person who has common knowledge in the field that includes the present invention. At this time, the dosage form may be a type of solution, suspension or emulsion in an oil or aqueous medium, or a type of extract, powder, granular, tablet or capsule. The composition according to the present invention may include a buffer solution that is dissolved with an adequate amount of salt or pH regulator in order to maintain a physiological activity of an effective component as efficiently as possible. In addition, the composition according to the present invention may be administrated by further including a dispersing agent or stabilizer in order to effectively apply the effective component according to the present invention.

The suited pharmaceutical acceptable carrier and pharmaceutical preparation are described in more detail in Remington's Pharmaceutical Sciences (19th ed., 1995).

The pharmaceutical composition according to the present invention may be orally or parenterally administrated. When administrating parenterally, it may be administrated by a type of intravenous infusion, subcutaneous transfusion, muscular injection, intraperitoneal infusion, transdermal administration, and the like.

The suitable dosage of the pharmaceutical composition according to the present invention may vary depending upon the factors, such as a way of formulation, a way of administration, a patient age, a patient body weight, a patient sex, a condition of patient, an administration time, an administration route, an elimination speed, sensitivity to the reaction, and the like. A doctor who is skilled generally can easily decide and prescribe the effective dosage for the desired prevention or treatment, and the dosage may be decided by dividing into one time or several times per a day. According to the preferable embodiment according to the present invention, the dosage per a day of the pharmaceutical composition according to the present invention is to administrate 1 mg to 150 mg of dapsone per 50 kg of object per one time, and preferably 50 to 100 mg of dapsone.

According to another embodiment of the present invention, the present invention is to provide a method for controlling the cell aging, in which the method includes applying the composition including dapsone as an effective component to the aging cell.

In addition, the present invention is to provide a method for extending lifespan of object, in which the method includes applying the composition to the object.

According to the specific embodiment of the present invention, the aging cell is an animal cell, preferably a mammalian cell, more preferably a human cell, and most preferably a human fibroblast.

In addition, the object is an animal, preferably a mammalian and *C*. *elegans*, and most preferably human and Caenorhabiditis elegans.

The duplicated content in the method according to the present invention and the composition according to the present invention as disclosed above will not be described in order to avoid the complexity of the description. In addition, the technical and scientific terms that are used in the description have the meanings similar to the meanings that are generally understood by the person who has general knowledge in the art unless they are not particularly defined.

### [Advantageous Effects]

The present invention relates to a composition for controlling aging and/or extending lifespan. More specifically, the present invention relates to a composition including dapsone as an effective component for controlling aging and/or extending lifespan, in which when applying with the composition according to the present invention, the effect of controlling aging or extending lifespan are excellent. Specifically, the administration of dapsone (DDS) leads to increase lifespan that is not dependent upon *deaf-16* in *C. elegans*, delay the aging process, and reduce the activities of mitochondria complex V protein and cytoplasm dual oxidase 1 (*Duox 1*). In addition, it is identified such that dapsone may maintain an oxygen uptake rate to be low in *C. elegans* thereby reducing ROS generation. In addition, the administration of dapsone leads to reduce ROS generation by paraquat that is an oxidative stress-inducing agent by treating with dapsone and decrease the sensitivity by paraquat. Moreover, it is suggested such that the target material of dapsone is a pyruvate kinase through the molecular and biochemical analysis, and the structural prediction. In addition, there are effects such that NOX expression is reduced and the reduced function of mitochondria is recovered by dapsone in a human fibroblast. According to the above comprehensive results, dapsone has an excellent effect for increasing lifespan in a level of object and cell.

### [Description of Drawings]

The above and other objects, features and advantages of the present invention will become apparent from the following description of preferred embodiments given in conjunction with the accompanying drawings, in which:

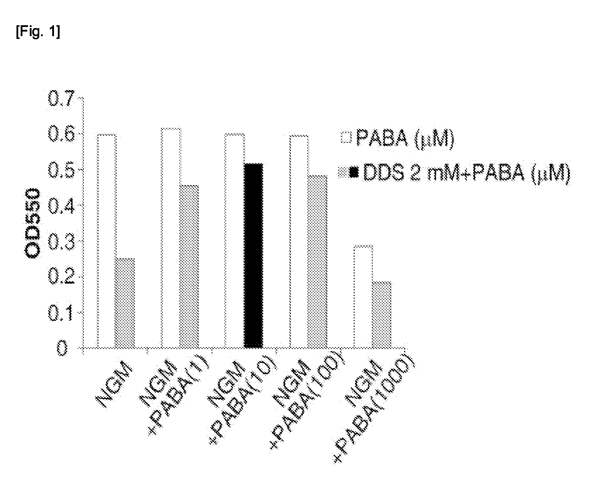

Fig. 1 is a graph showing the growth degree of bacteria by treating with PABA. At this time, the growth degree of bacteria was identified in the control group that was treated with 2 uM of PABA alone and the group that was treated with PABA and 2 mM of DDS, respectively. The effect for restricting food was prevented by suppressing OP50 proliferation by DDS. OP50 bacteria were treated only with PABA (2 uM), or were treated with PABA and DDS at the same time for 36 hours.

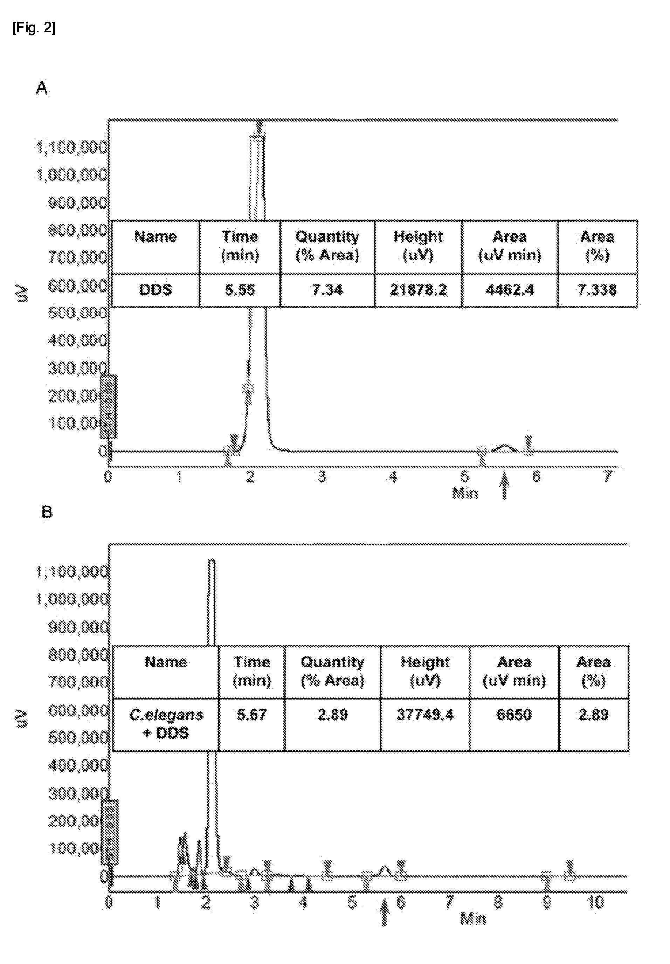

Fig. 2A and 2B are graphs showing DDS intake amounts in *C. elegans* that were administrated with DDS. More specifically, they are the results of chromatograms of the worms including DDS by using HPLC; DDS was measured in the worm that feeds on OP50 bacteria treated with DDS; 10 uM of DDS (Top A) was injected into HPLC as a comparative control; and the worm that feeds on OP50 treated with DDS was measured (Bottom B); they are compared to each other. The parenthetic tables that are shown in the graphs indicated the detailed information of DDS peaks.

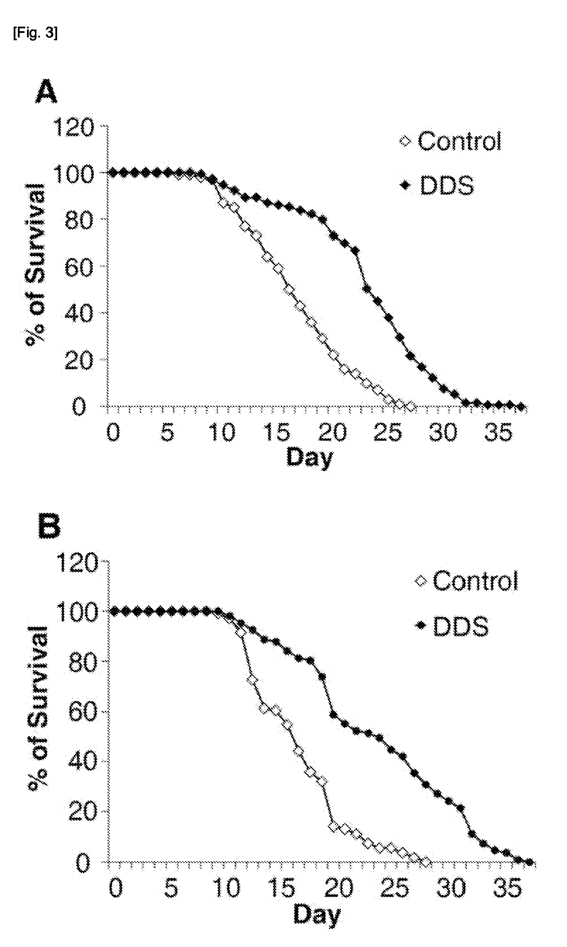

Fig. 3A and 3B are the growth curves of *C. elegans* that were administrated with dapsone. Fig. 3A is the graph showing the growth curve (survival rate) of *C*. *elegans* that were administrated with DDS (2 mM, n=129) in the whole life; and Fig. 3B is the growth curve of *C*. *elegans* that were administrated with DDS (2 mM, n=107) after finishing the generation (Adolescence, L4 stage).

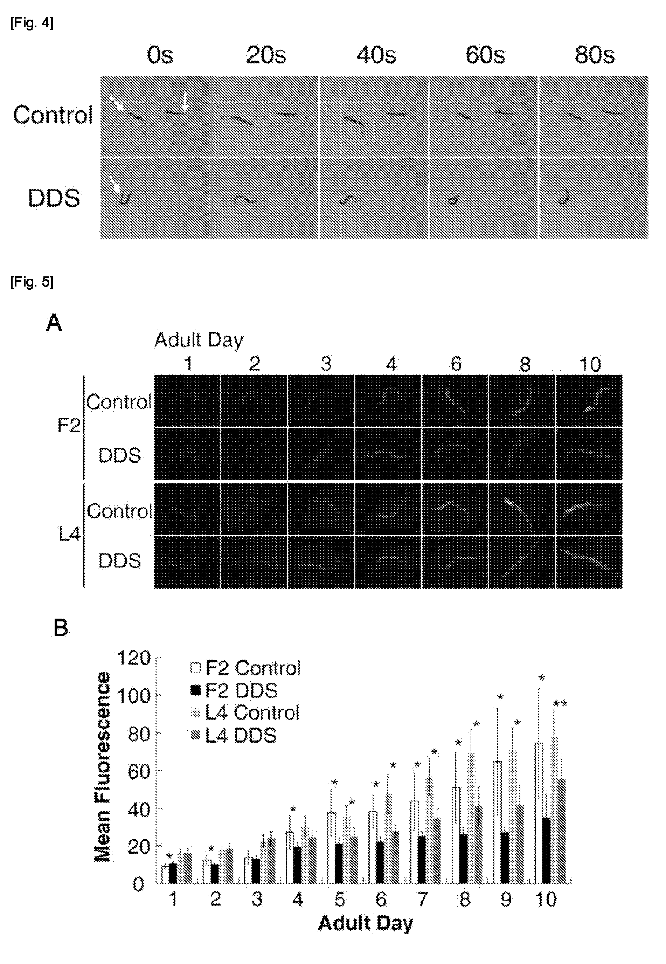

Fig. 4 is the vitality(movements) of worm at 23 days after taking with DDS or not taking. It shows the results for taking pictures per 20 seconds after administrating. At this time, the arrows indicate the initial positions of worms.
Fig. 5 is the result of delaying the aging process by DDS, and Fig. 5A is the photograph showing a chemiluminescent of lipofuscin in a small intestine of worm that fed on DDS after L4 stages and in the whole life. At this time, the numbers mean the days after becoming an adulthood; F2 means 2-generation after administrating with DDS; and L4 means the administration of DDS after L4 generation. Fig. 5B is the graph showing the quantitative difference of lipofuscin accumulation and 30 more than of worms were observed per a day. An error bar indicates a standard deviation, * indicates p<0.0001 and ** indicates P<0.001 (Mann-Whitney t-test).

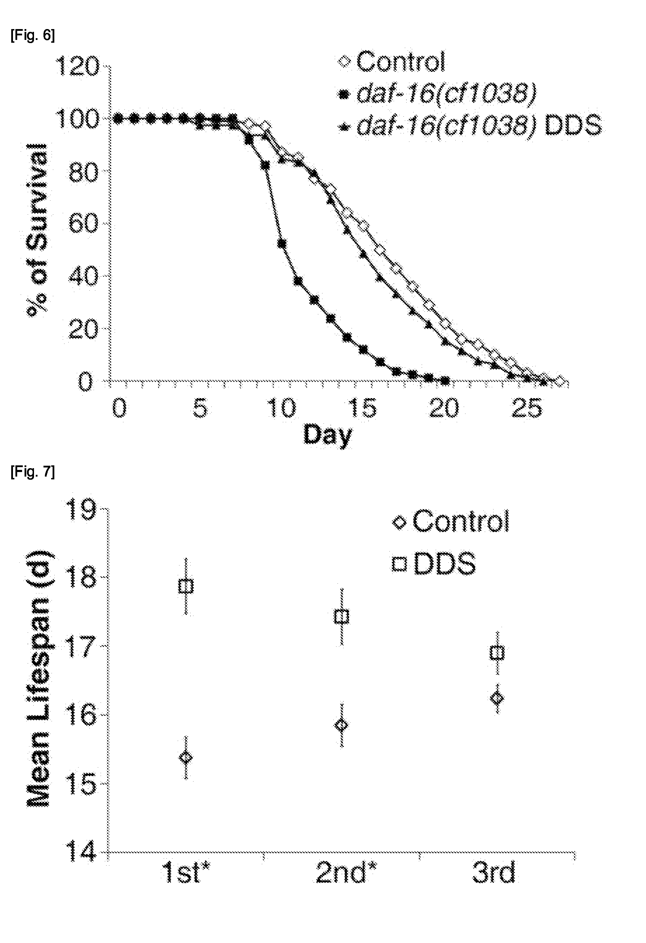

Fig. 6 is a graph showing the survival rates of *daf-16 (cf1038)* mutant worms (n=100) that fed on *E. coli* including PABA and *daf-16 (cf1038)* mutant worms that fed on *E. coli* (n=78 and 84, respectively) including PABA and 2 mM of DDS, and it could be shown that the effect of DDS does not depend upon *daf-16*.
Fig. 7 is a graph showing the growth curves of *C. elegans* that fed on the bacteria(*E*. *coli*) being defective in *folP* or not being defective in *folP* that is a target material for DDS. At this time, it could be shown that in the case of *C. elegans* that fed on the bacteria being defective in *folP(*normal *E.coli)*, the lifespan was extended. In the case of two results from three experiments, the lifespan was significantly extended.

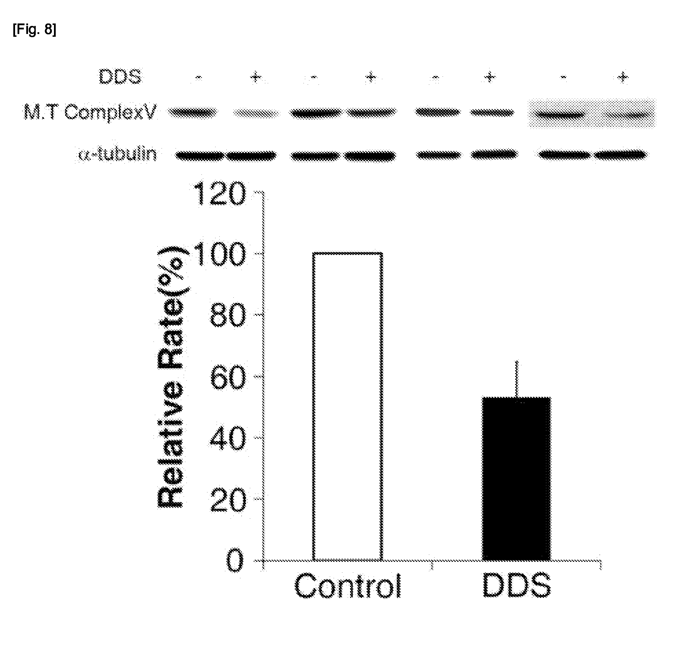

Fig. 8 is a graph showing the results obtained by isolating mitochondria and then performing Western Blot in order to measure the amount of mitochondria complex V. α-tubulin was used as a comparative control and the results were statistically calculated by using the mean and deviation obtained by repeating four times of experiments.

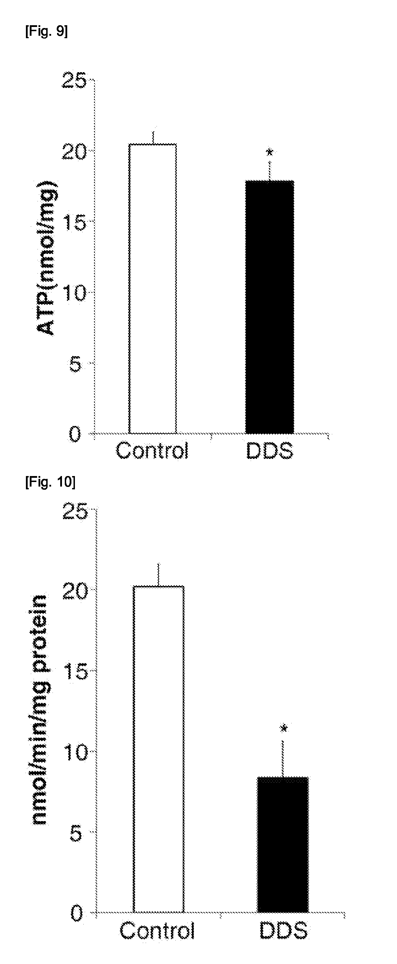

Fig. 9 is a graph showing the amount of ATP in *C. elegans* that were treated (administrated) with DDS.
Fig. 10 is a graph showing the oxygen uptake rate of *C. elegans* that were treated (administrated) with DDS. At this time, the experiment was repeated four times and the mean of them was calculated.

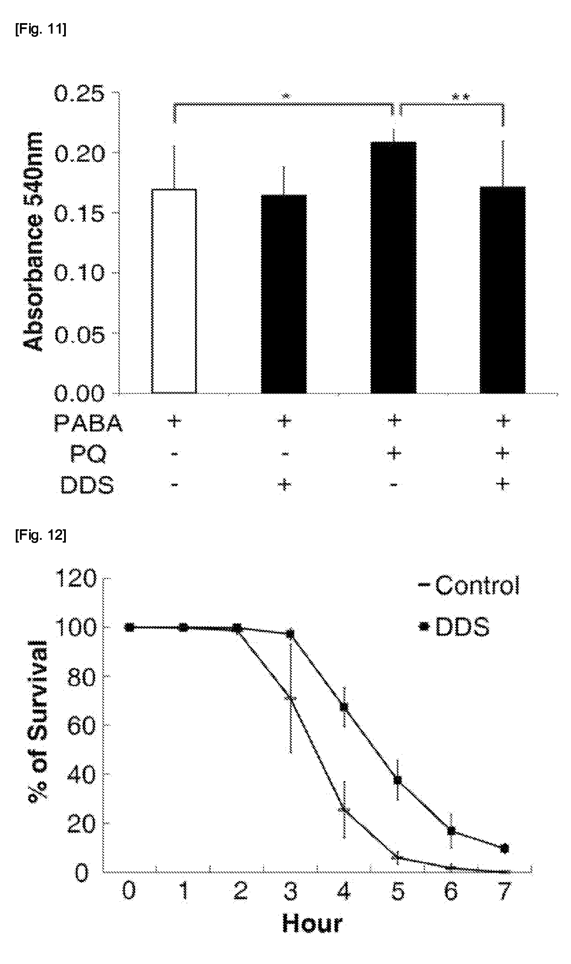

Fig. 11 is a graph showing the results, in which H₂O₂ generated by PQ was suppressed by administrating with DDS. H₂O₂ generation was measured by using a plate reader at 540 nm of observance by using AmplexRed. (*: comparing with a control that was not treated with PQ, **: comparing with the group that was treated with PQ, but not DDS)
Fig. 12 is a graph showing the survival rates(survival curves) of worms that were treated with DDS or not treated with DDS in a NGM liquid medium containing PQ (250 mM) for 7 hours. At this time, the two groups were treated with PABA and the lifespan was measured until the death of the worms (*p*<0.0001).

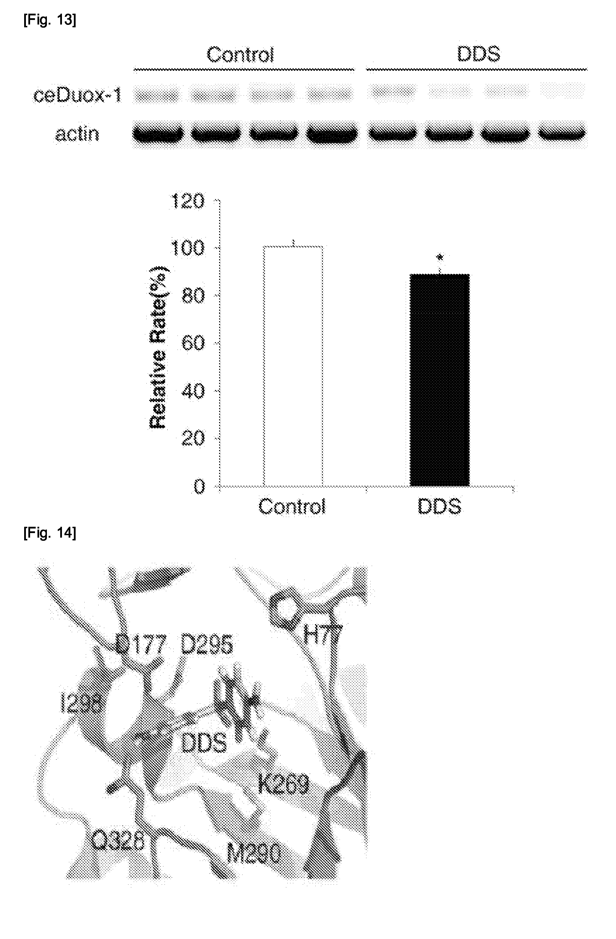

Fig. 13 is the results of measuring the amount of *ceDoux1(a C. elegans* NADPH oxidase*)* mRNA in the worms that fed on DDS or not fed on DDS by using RT-PCR. At this time, actin was used as a comparative control and the results were obtained by repeating four experiments and calculating the mean thereof (*P<0.005).
Fig. 14 shows a structural modeling of DDS and PK (PEP binding activity region).

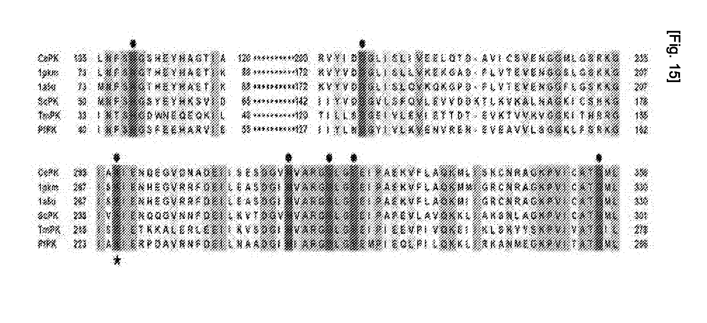

Fig. 15 shows the pyruvate kinase sequences of each different species. The red and dot indicate the potential reside (PEP binding activity region) that is interacted with DDS. Abbreviations: CePYK-1, *C. elegans* PYK-1; CePYK-2, *C*. *elegans* PYK-2; Sc, *S. cereviciase*; Tm, *T. maritime*; Pf, *P. furiosus*; 1 pkm, cat PK; and 1a5u, rabbit PK. The residue K246 that is indicated with an asterisk (*) is one of PEP binding sites.

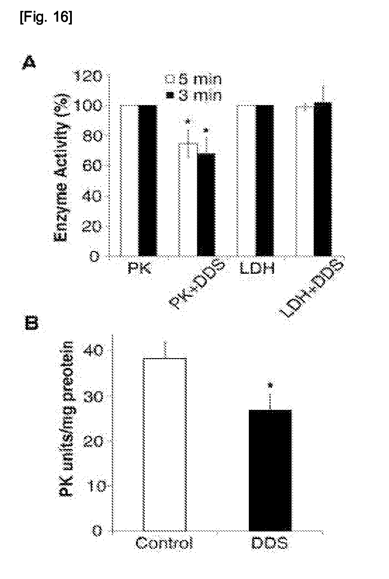

Fig. 16A and 16B are the results of 'PK suppression' by DDS *in vitro* (16A) and *in vivo* (16B). PK activity was analyzed through the decrease degree of absorbance at 340 nm by using the change result from pyruvate and NADH to lactate and NAD by lactate dehydrogenase (LDH). The pyruvate and ATP used for the above process were produced from phosphoenolpyruvate (PEP) and ADP by PK. The analysis *in vitro* was measured by adding directly DDS along with PK to the above reaction mixture and the analysis *in vivo* was measured by adding worms that fed on DDS.

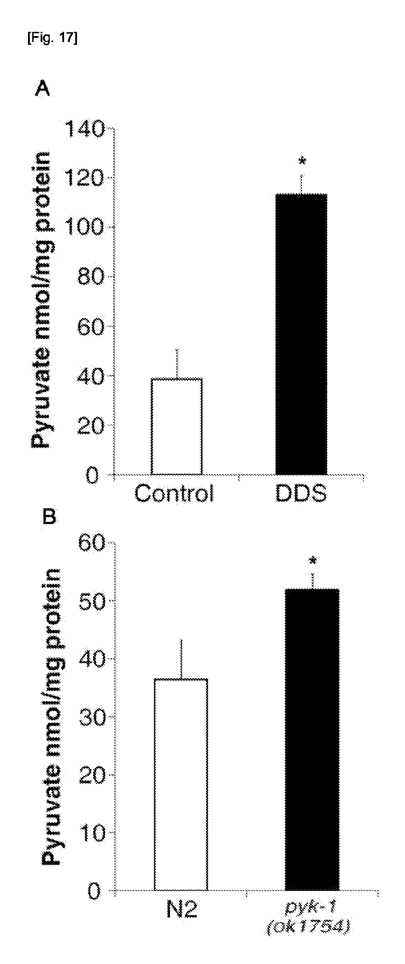

Fig. 17A shows the increase of pyruvate content in *C*. *elegans* that were treated (administrated) with DDS and Fig. 17B shows the increase of pyruvate content in *pyk-1* (ok1754) mutant worms that were not treated with DDS.

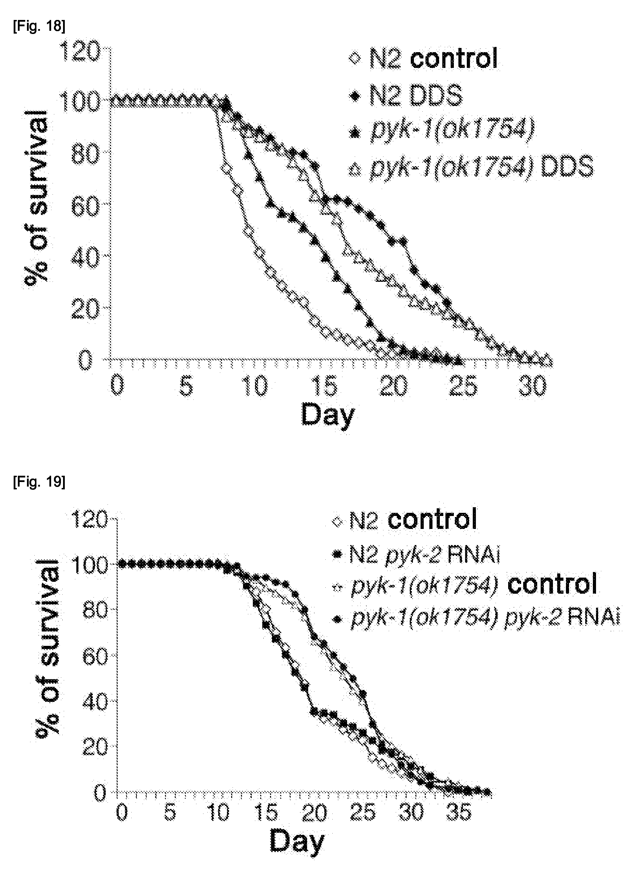

Fig. 18 is a graph showing on extending lifespan of *pyk-1* (ok1754) mutant animals and shows the results of increasing lifespan of *pyk-1* (ok1754) mutant animals that were not treated with DDS. N2 and ok1754 mutant fed on OP50 bacteria in the medium that includes only PABA or PABA+DDS.
Fig. 19 is a graph showing that *pyk-2* RNAi does not affect the lifespan of wide-type N2 and *pyk-1* mutant animal.

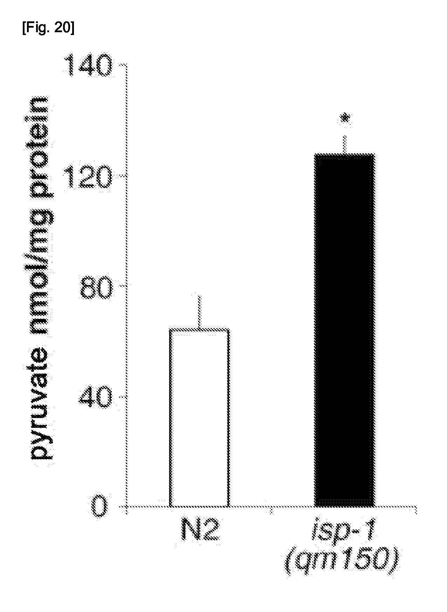

Fig. 20 shows the increase of pyruvate content in *isp-1* mutant worms (*P<0.001).

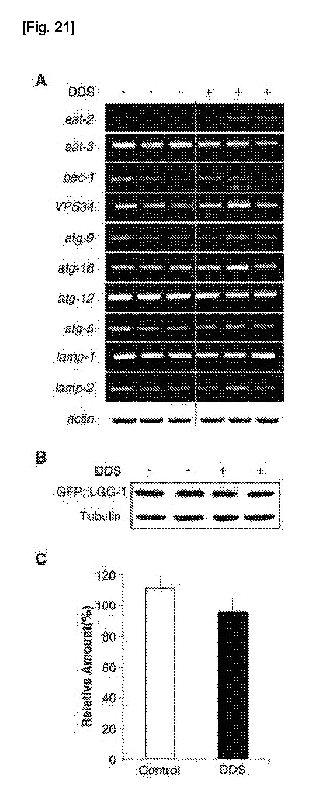

Fig. 21A and 21B are the results showing that DDS does not affect an autophagy in *C*. *elegans*. Fig. 21A is to identify mRNA of gene that is related to the autophagy in the worms that fed on DDS, or not fed on DDS by using RT-PCR and Fig. 21B is to perform by using an immune analysis in the transformated *C*. *elegans* having GFP::Igg-1 that is a marker of an autophagy. It could be shown that there is no meaningful difference in the worms that fed on DDS through the immune blot. All the results are summarized from three independent experiments.

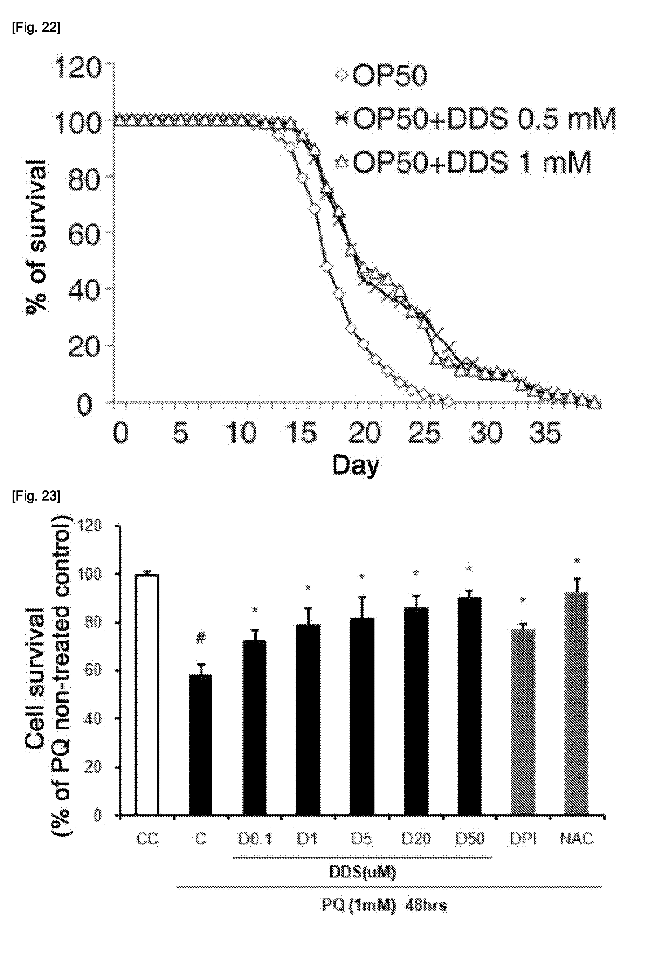

Fig. 22 is the result showing that even if the concentration of DDS is low, there is a similar effect for extending lifespan. The survival curves in the worms that fed on 0.5 mM of DDS and fed on 1 mM of DDS have the almost similar effects for extending lifespan.
Fig. 23 is a graph showing the effect of DDS to the survival ability of human diploid fibroblasts (HDFs) that was treated with PQ, in which DDS was treated to the human diploid fibroblasts for 3 hours and 1 mM of PQ was treated for 48 hours. The above cell was cultured by adding Cell Counting Kit-8 for 48 hours and then the survival ability of cell was measured. In addition, DPI (5 uM) was pretreated for 30 minutes and NAC (2 mM) was pretreated for 3 hours, and then 1 mM of PQ was treated as a positive control. The result was indicated as a control rate (%) by standardizing with a control. Three experiments were performed and the statistical significant was expressed as # and * significantly different p<0.005 (#: Control (CC) that was not treated, *= Control (C) that was only treated with PQ).

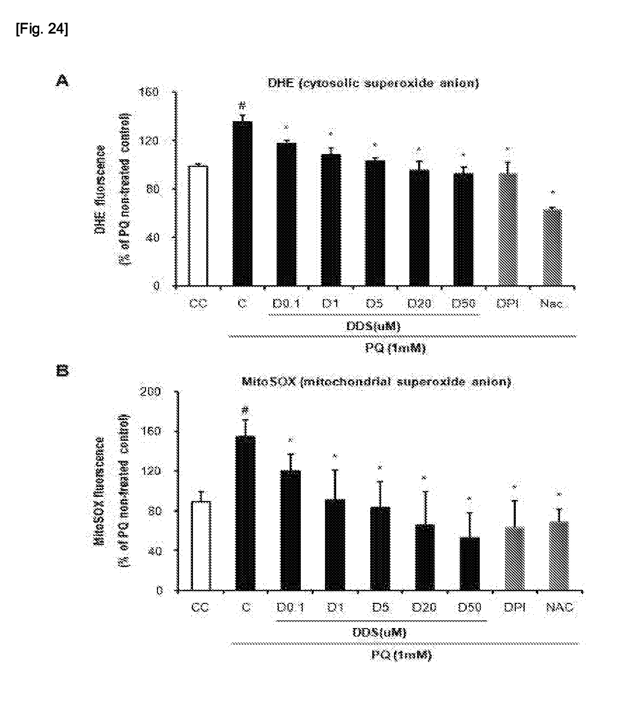

Fig. 24A and 24B are the graphs showing the effect of DDS on the generation of the cytosolic or mitochondria superoxide anion that is induced by PQ, in which the human diploid fibroblast was cultured at 96 well plate for 24 hours and then was pretreated with NAC (2 mM) for 3 hours. The cell was treated with 1 mM of PQ for 30 minutes, and then was cultured at 37 °C for 15 minutes after adding oxidant-sensitive fluorescent dyes (A) DHE (5 uM, cytosol specific) and (B) MitoSOX (5 uM, mitochondria specific), respectively. For identifying the change of fluorescence, the excitation and emission wavelength of DHE were measured at 515 and 590 nm and MitoSOX was measured at 520 and 580 nm by using a multiwell plate reader. The results were indicated by calculating the results of three experiments as a control rate (%). The statistical significant was expressed as *, # p<0.005; ** p<0.05.

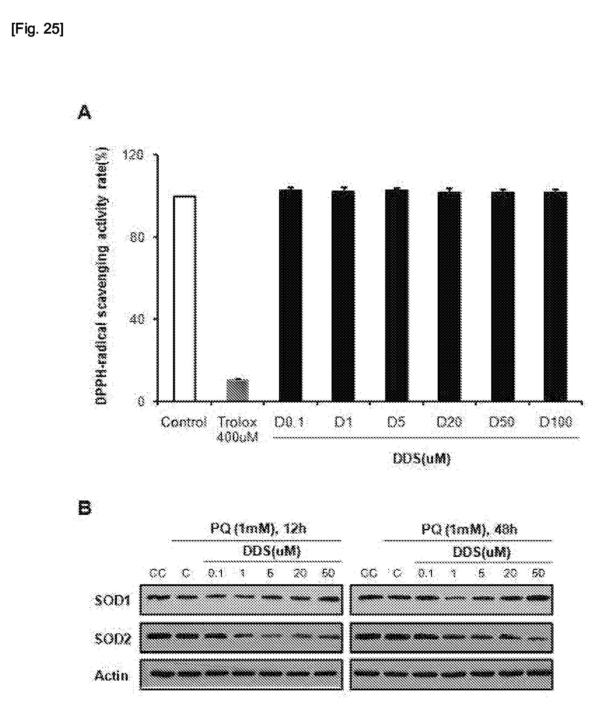

Fig. 25A and 25B are the results of measuring DPPH-radical scavenging activity of DDS *in vitro*, and show the effects of DDS on the change of protein amount of SOD1 and SOD2 that are enzyme for removing the superoxide anion generated in HDF that was treated with PQ. Fig. 25A is a graph showing the rate of suppression of DPPH (200 uM) radical as the effect of DDS having the radical scavenging activity in Cell-free system (*in vitro*). Trolox that is well known as a water-soluble vitamin E was used as a positive control. Fig. 25B shows the expressions of (Cu/Zn-) SOD1 and (Mn-) SOD2 in the human diploid fibroblast that was pretreated with DDS for 3 hours and was treated with 1 mM of PQ for 12 hours or 24 hours by using Western Blot analysis.

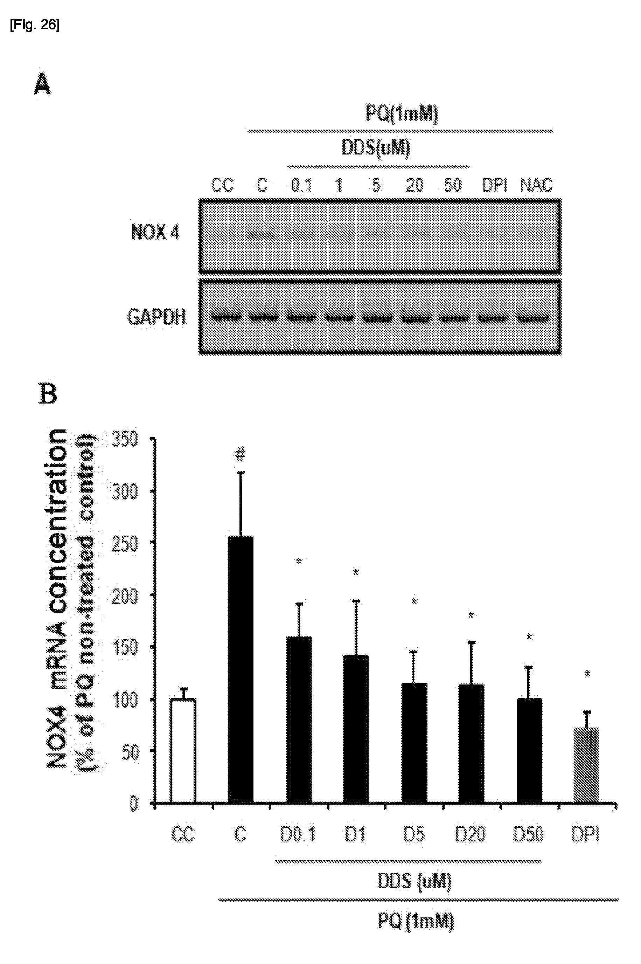

Fig. 26A and 26B show the results of DDS on NOX4 mRNA increased by PQ in the human diploid fibroblast. For Fig. 26A, the human diploid fibroblast was pretreated with DDS, DPI (5 uM) and NAC (2 mM), and then was treated with PQ (1 mM) for 5 minutes. The amount of NOX4 (HDF specific) mRNA, which is a source for generating a cytosolic superoxide anion, increased by PQ was measured. For Fig. 26B, it was quantitived as % rate as compared with a control (non-treated human diploid fibroblast).

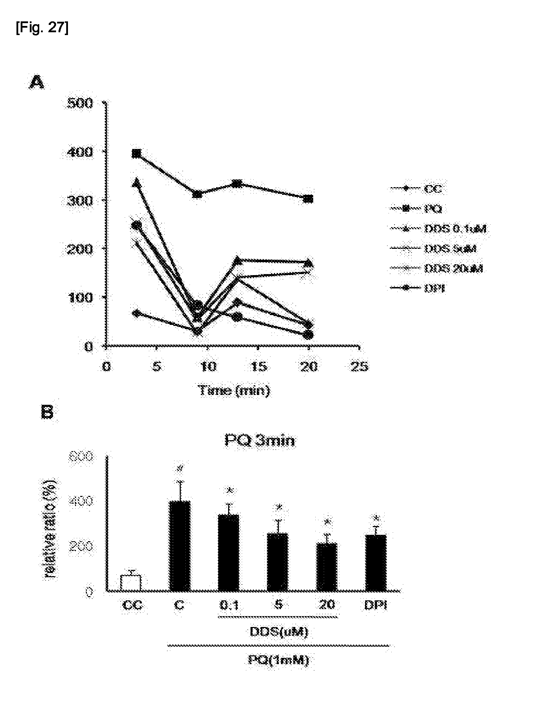

Fig. 27A and 27B show the effects of DDS on PKC activity depending upon calcium that is induced by PQ in the human diploid fibroblast, in which the effect of DDS on the change of calcium amount that is induced by PQ in the human diploid fibroblast was shown. HDF cell was cultured at 96-well plate and the calcium in cell was measured by using Fluo-4AM. The cell was pretreated with DDS (27A) and 5 uM of DPI, and then was treated with 1 mM of PQ (27B).

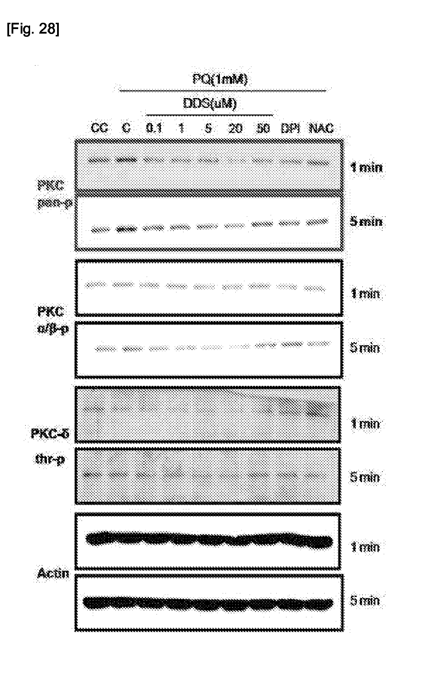

Fig. 28 shows the results by measuring PKC phosphorylation that is induced by PQ using Western Blot, in which PKC phosphorylation was analyzed by collecting the cells that was pretreated with DDS and then was treated with 1 mM of DDS for 1 minute or 5 minutes (Statistical Significant was compared to # p<0.05 CC and * p<0.05 C).

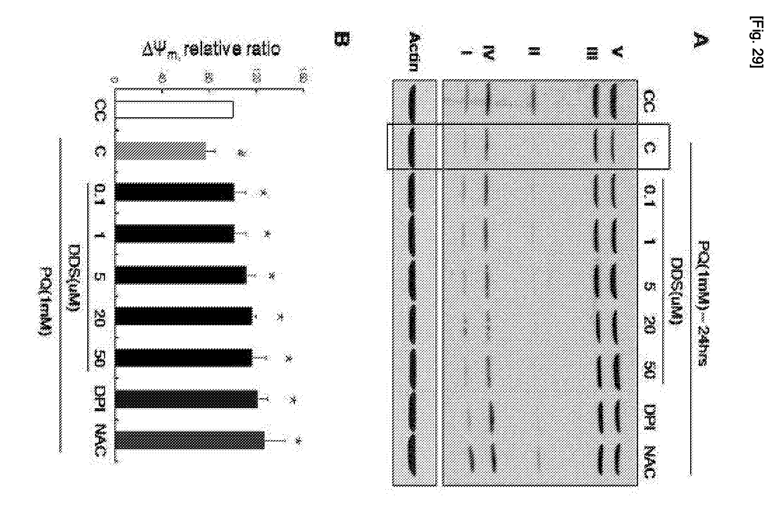

Fig. 29A and 29B show the effects of DDS on the mitochondria change that is induced by PQ in the human diploid fibroblast. In order to identify the effect of DDS on the mitochondria toxicity that is induced by strongly treating with 1 mM of PQ for a long time, the inventors observed various changes that are induced by PQ, such as the decreased protein amount of mitochondria constituent complex, the decreased membrane potential of mitochondria, and the like. For Fig. 29A, the human diploid fibroblast was pretreated with DDS, DPI and NAC, and then was treated with 1 mM of PQ for 24 hours. The protein amount of mitochondria constituent complex was measured by using Western Blot using Complex I (20 kDa ND6 subunit), Complex II (30 kDa FeS, non-heme iron protein, SDHB), Complex III (47kDa core protein 2), Complex IV (18 kDa subunit IV), Complex V (55 kDa subunit a ATP synthase), and the like. Fig. 29B shows the effect of DDS on mitochondria membrane potential that is induced by PQ in the human diploid fibrobast. The membrane potential (ΔΨm) of mitochondria used DiOC₆. The human diploid fibroblast was pretreated with DDS, DPI and NAC, and then was treated with 1 mM of PQ for 24 hours. Since then, the above fibroblast was treated with 40 nM of DiOC₆ for 15 minutes. The results were measured by using Cary Eclipse Fluorescence Spectrophotometer (Varian, California, USA) and the excitation wavelength and emission wavelength were measured at 480 nm and 520 nm, respectively.

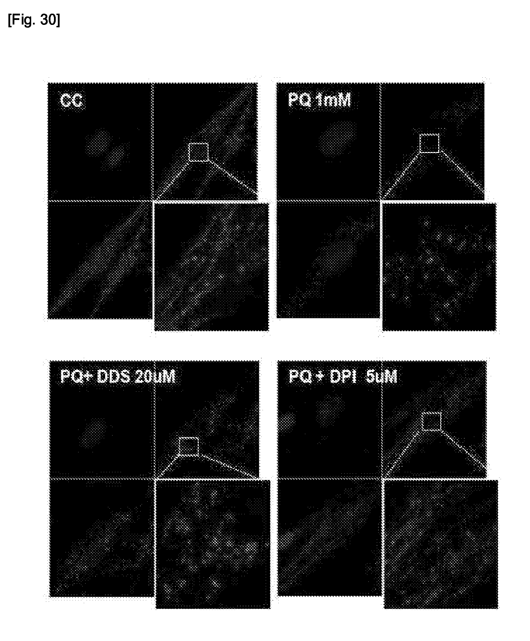

Fig. 30 shows the results obtained by staining using Mitotracker Red and then visualizing using Confocal Microscopy in order to analyze the structural change of mitochondria. Like the above experiment, the human diploid fibroblast was treated with 1 mM of PQ (for 24 hours) and DDS (or DPI), and then was covered with a cover glass. The first photograph indicates the stained mitochondria of the non-treated human diploid fibroblast as a control, the second photograph indicates the stained mitochondria of the PQ-treated human diploid fibroblast, the third photograph indicates the stained mitochondria of the human diploid fibroblast that was pretreated with DDS and then was treated with PQ, and the final photograph indicates the stained mitochondria of the human diploid fibroblast that was treated with DPI and PQ together (by using Confocal process). The values of (A, B, C and D: x 4000) is means ± SEM (n=3), # is p<0.005 relateive CC; and * is p<0.005 relative C.

### [Best Mode]

Hereinafter, the embodiments of the present invention will be described in detail with reference to accompanying drawings. The embodiments are given by way of illustration only for describing the present invention in more detail, and it will be understood by the person who has general knowledge in the art such that the point of the present invention will not be limited to the above embodiments according to the point of the present invention.

### <Example>

### I. Effect of Dapsone on Lifespan of Object

### Example 1. Experiment Materials

*C. elegans* was used as a model animal in order to identify the effect of dapsone on lifespan of object. A descent of *C. elegans* used for the experiment is a wide-type N2 (Bristol) and the mutant species, such as *daf-16* (cf 1038), *clk-1 (e2519)*, *isp-1* (*qm150*) and *adls2122* [GFP::*Igg-1*, *rol-6* (*su10060*)].

### Example 2. Effect of Dapsone on the Extending of Lifespan

In order to identify the effect of dapsone on the extending of lifespan, the experiment was performed in the following order. At this time, since dapsone is not a water-soluble material, *E*. *coli* was cultured in the medium including dapsone and then was used as a feed for *C. elegans*. In addition, the growth of *E. coli* was increased by adding 10 uM of PABA (*para*-aminobenzoic acid) to the medium solution thereby preventing the caloric restriction of *C. elegans*. The administration of dapsone was started after L4 stage that almost finishes the generation, and the experiment was performed as follows under the condition of 20 °C in order to provide the optimal growth environment:
1) A parent *C*. *elegans* of L4 stage was transferred to the medium containing dapsone and PABA, and the progeny of first generation was transferred to the new medium after four days to lay an egg for 4 to 5 hours. Ten of the progenies of second generation after hatching were continuously transferred to the new medium until they do not lay an egg and then the lifespan was measured.
2) Ten of the parent *C*. *elegans* of L4 stage were transferred to the medium containing dapsone and PABA and were continuously transferred to the new medium until they do not lay an egg to measure lifespan.

*C. elegans*, which die because of getting on in years, was excluded from the statistical analysis. When touching *C*. *elegans*, they do not move so that they were regarded as dead. The significant difference of statistical average lifespan used Log-rank test [47].

### Example 3. Measurement of Chemiluminescent of Lipofuscin

The chemiluminescent of *C*. *elegans* was taken a photograph by exposing for 800 msec through 525 nm Band-Pass Filter. The size of each photograph was only adjusted by using Adobe Photoshop CS2.

For measuring the amount of Chemiluminescent in a small intestine, the brightness of pixels in a certain region was measured by Image J 1.35. At least 30 of *C. elegans* were measured every day and then the mean and standard error were calculated.

### Example 4. DDS Detection by using HPLC

DDS was measured by using the method of Kwadijk et al. [48]. In order to prepare the sample from DDS-treated *C. elegans* (*C. elegans* grown in the medium containing DDS), 0.26 g of *C*. *elegans* was added to 1 mL of mixed solution of acetone and glutathione (5 mg/mL, water:methanol 1:10) and then completely mixed. After centrifuging at 2700 g for 2 minutes, the solution was evaporated with Speed Back, and the residue was dissolved in 80 uL of the mixed solution of HPLC eluent and acetone (18:5, v/v). The samples that were stored by dividing each 20 uL and the standard material, 10 uM of DDS for comparing, in which the concentration of standard material is known, were injected into HPLC column. The HPLC system is constituted of JASCO HPLC pump PU-980 and JASCO UV-975 detector. C18 150 x 4.6 mm (5 um) column was used for isolating the sample and the speed for passing was to be 1.0 mL/min. DDS used the dissolving solution that is composed of water:acetonitrile:glacial acetic acid:triethylamine (80:20:1.0:0.05, by volume) and the detection of amount was performed at 295 nm (285 nm emi/340 nm exi).

### Example 5. Isolation and Preparation of Mitochondria

*C. elegans* was isolated and collected by using dense sucrose (Centrifugation) and was washed with S buffer solution to remove a contaminant. In order to isolate mitochondria, *C. elegans* was twice homogenized in MSM buffer solution (220 mM mannitol, 10 mM sucrose, 5 mM MOPS, pH 7.4, with KOH) containing 0.2 % of BSA at 5000 rpm for 20 seconds by using Precellys24 Homogenizer (Medinova) and Glass bead. The residue and non-homogenized *C*. *elegans* were removed fro the homogenized *C. elegans* through a centrifuge (380g, 5 min) by using MSM buffer solution. The precipitate of homogenous substance that was centrifuged at 4500 g for 5 minutes, i.e., mitochondria were prepared. The rest was prepared as a part of cytoplasm. After the freezing and thawing of the isolated mitochondria and a part of cytoplasm were repeated, they were treated with 0.8 % of CHAPS before measuring the amount of mitochondria complex V [49]. The quantitation of protein was performed by using BCA protein detection kit (Pierce).

### Example 6. Western Blot Analysis

The lysates of *C. elegans* grown under appropriate condition (containing PABA alone or PABA and dapsone) were prepared. The mitochondria and cytoplasolic fractions were boiled in the sample buffer solution (50 mM Tris-HCl (pH 6.8), 2 % SDS, 0.14 M 2-mercapto ethanol, 10 % glycerol and 0.001 % bromophenol blue). And then, the mitochondria and cytoplasolic fraction prepared from the above process were isolated by using an electrophoresis. After transferring to a nitrocellulose, they were analyzed by using Western Blot using MS601 (subunit α of F1 of ATP synthase, Oxphos Complexes detection kit, Mitoscience, 1:1,000) and α-Tubulin (Sigma; 1:2,000) antibody. The result was detected by using ECL (Pierce) and a horseradish peroxidase-conjugated secondary antibody.

### Example 7. Measurement of ATP amount

The amount of ATP was measured by slightly modifying the method as mentioned above [38]. In order to analyze the total amount of ATP, *C. elegans* was cultured by feeding only on PABA or by feeding on PABA and dapsone, and then was washed with S Basal buffer solution (0.1 M NaCl/0.05 M potassium phosphate buffer, pH 6.0) to remove a contaminant and prepare a sample for measuring. The sample was ultimately adjusted to be 100 ul and then froze at - 80 °C. *C. elegans* that was frozen was immediately heated for 15 minutes to erupt ATP and then maintained at a cold condition. Since then, the residue of *C*. *elegans* was removed by using a centrifuge (at 15,000 g for 5 minutes). The amount of ATP was measured by using the reagent of bioluminescence assay kit CLS II (Roche Molecular Biochemicals) and Luminometer (available from PerkinElmer company). The amount of ATP was standardized with the amount of protein.

### Example 8. RT-PCR of Gene related to Oxidative Stress

Total RNA was isolated by using TRI reagent available from MRC Company and first cDNA strain was synthesized by using Oligo-dT and Superscript II Reverse Transcriptase (available from Invitrogen Company). The gene amplification (PCR) was performed by using PCR Master Mix (Genenmed) and as the base sequence of the primer, *ceDuox1*; 5'-CTTCACACCGTTGGACATTG-3' (Forward Primer) and 5'-GAAGATGTGTGAGCCGGAAT-3' (Reverse Primer), *actin* ; 5'-TCGTAGGACTTCTCGAGGGA-3' (Forward Primer) and 5'-ATGTTGCCGCTCTCGTAGTT-3' (Reverse Primer) were used.

In addition, the gene related to the autophagy used the primer as follows: *eat2:* Forward Primer (5'- GCAAATTCCCCATGGTACAC -3') and Reverse Primer (5'- CATGGAAAGTGAGCACGAGA -3'); *eat3*: Forward Primer (5'-AGGCTGCATCAGAACGTCTT -3') and Reverse Primer (5'-TGTTTGTGCTCTGGATCTGC -3' ); *bec1*: Forward Primer (5'-CAAAGAAGGCCAGATTCAGC -3') and Reverse Primer (5'-CGTTGTCGGATGGTTTTCTT -3'); *let-512* /*VPS34*: Forward Primer (5'-TATGCGAGTCTCCACGTCAG -3') and Reverse Primer (5'-CCAATCGATCCTTTGCTTGT -3'): *T22H9.2 (atg9)*: Forward Primer (5'-CACTTCAACGAGCTTGACCA -3') and Reverse Primer (5'-GTGATGACGTGTTCCACCTG -3') ; *atgr-18 (atg-18)*: Forward Primer (5'-CAGGAAGCACTGACACTGGA -3') and Reverse Primer (5'-AAAGAGCCGATGTCCATTTG -3' ); *Igg-3 (atg-12)*: Forward Primer (5'-TGAAATTGCGAAAACTGCTG -3') and Reverse Primer (5'-GTAGGCCGGTGTAATGCTGT -3'); *atgr-5 (atg-5)*: Forward Primer (5'-CGAATTTGCACACATTCCAC -3') and Reverse Primer (5'-TCCGTTGAGGATGATGATGA -3'); *lamp1*: Forward Primer (5'-CAACGCTTACAAGTGCTCCA -3') and Reverse Primer (5'-ACGACGATTGGGACAACTTC -3'); *lamp2*: Forward Primer (5'-GGCCAAAAGAAACTTGTCCA -3') and Reverse Primer (5'-TTTCGTCATTGGAAGCTGTG -3').

### Example 9. Measurement of Hydrogen Peroxide by using Amplex Red Assay

The effect of dapsone on ROS scavenging was measured by using Amplex Red hydrogen peroxide/peroxygenase detection kit (Molecular Probes, Eugene, OR) [39] that were previously described in order to measure the effect of dapsone on ROS scavenging. Simply put, 150 *C. elegans* that fed only on PABA or fed on PABA and dapsone was treated with 250 mM of paraquat for 1 hour. 100 *C*. *elegans* were adjusted to be 50 ul at 96-well plate and then 50 ul of Amplex Red (200 uM) was added to each well. After maintaining at 22 °C for 90 minutes, the amount of hydrogen peroxide was measured at 540 nm of absorbance by using a plate reader (Tecan, infinite 200).

### Example 10. Test for Oxidative Stress Resistance by Dapsone

*C. elegans* at 5 days after administrating dapsone for second generations was used for testing an oxidative stress resistance by dapsone. 250 mM of paraquat was dissolved in M9 solution and then 20 *C. elegans* was immersed into the above solution. The number of living *C. elegans* was measured per an hour.

### Example 11. Measurement of Oxygen Uptake Rate

The oxygen uptake rate was measured by using the method disclosed in the theses (50, 51) using Clark-type oxygen electrode (782 Oxygen Meter, Strathkelvin Instruments, Glasgow, UK). *C. elegans* was growth in NGM agar medium that contains only PABA or PABA and DDS, fed on OP50, and then washed by using S-basal buffer four times to wash a contaminant. Approximately 1500 *C. elegans* in 200 uL of S-basal buffer were added to Mitocell chamber with Clark-type oxygen electrode to measure the oxygen uptake rate for several minuets. A sample was carefully taken from the chamber and then twice homogenized at 5000 rpm for 20 seconds by using Precellys24 homogenizer with glass bead. The sample was centrifuged at 15000 g for 20 minutes and the protein in the supernatant was quantified by using BCA protein quantification kit.

### Example 12. DDS Binding Modeling

DALI search [52] was performed by using *Streptococcus pnuemoniae* (PDB ID; 2VEG), Cat & rabbit pyruvate kinase (PDB ID; 1 PKM, 1A5U respectively), and the structure of dihydropterase synthase(DHPS) to obtain Z score 15.1 and 15.0. Both structures have RMSD value of 3.1 between 519 residues. DDS was initially modeled into the structure of DHPS's PABA binding site, according to the position of aminophenyl group of PABA, The structure of cat pyruvate kinase was superimposed to the DHPS. The final model of DDS was modified manually with the program COOT, regarding the steric hindrance potential residues of PK and DDS [53]. The potential binding beween PK residues and DDS was selected by using the model of DDS and cat pyruvate kinase structure and PK sequences of six different species were preserved.

### Example 13. Measurement of Pyruvate Content

The measurement of pyruvate content was performed by using Fluorescence-based assay kit (BioVision) was purchased according to its instructions. The measurement of pyruvate content was adjusted with total proteins and the pyruvate content exhibits as nmol/mg of protein. Three measurements were performed.

### Example 14. Measurement of Pyruvate Kinase Activity

Samples were homogenigized y b rupturing *C. elegans* with glass bead Precellys 24 homogenizer, twice for 20 seconds, at 5000 rpm in a 100 mM potassium phosphate buffer, pH 7.6, at 37 °C. A CHAPS was added to the homogenized sample to be 1% of the final concentration and then centrifuged at 15000 g for 20 minutes. The measurement of pyruvate kinase(PK) activity was performed at 37 °C by adding 300 uL of the reaction mixture contained 100 mM potassium phosphate buffer at pH 7.6 and 37 °C, 100 mM MgSO₄, 1.3 mM β-NADH, 5000 units/mL of lactate dehydrogenase (LDH), 2 mM adenosine diphosphate (ADP) and 17 mM phosphoenolpyruvate (PEP) into the sample. The change of oxidation degree of NADH was measured at 340 nm of absorbance. PK 1 unit was defined as the change into 1.0 umole of pyruvate per a minute at pH 7.6 and 37 °C. The protein quantification was measured by using BCA protein detection kit (Pierce) and the pyruvate kinase activity exhibits as units/mg of protein. Type I pyruvate kinase (Sigma) of rabbit muscle was used for *in vitro* experiment. After identifying whether or not DDS suppresses LDH activity, in order to exclude the suppression, PK was not treated to the control and pyruvate instead of PEP was treated.

### II. Experiment for Controlling Aging in the Level of Cell

### Example 15. Experiment Materials

Dapsone (4,4'-diaminodiphenylsulfone, hereinafter called as DDS) was obtained from Taeguk Chemical Company, and Paraquat, 2,2-diphenyl-1-picrylhydrazyl (DPPH), Trotox, iodonium (DPI) and N-acetyl-L-cystein (NAC) were obtained from Sigma. Cell Counting Kit (CCK-8) was purchased from Dojindo Laboratory Company (Japan). Reagent was purchased from MRC (Cincinnati, USA). The fluorescence materials, such as Dihydroethidium (DHE), Red, Fluo-4 AM, MitoTracker Red CMXRos and 3,3-dihyxyloxacarbocyanine (DiOC6) were purchased from Molecular Probes (Eugene, OR).

### Example 16. Evaluation of Survival Rate of Cell

The evaluation of survival rate of cell was performed by using Counting Kit (CCK-8) from Dojindo Laboratory Company (Japan). The human diploid fibroblast was cultured at 96-well plate for 24 hours before treating. The human diploid fibroblast was treated with DDS for 3 hours, was treated with 1 mM of PQ for 48 hours, was directly treated with 10 ul of CCK-8, respectively, and then cultured at 37 °C under the condition of 5% CO₂ for 3 hours. The absorbance was measured at 450 nm. The results were compared with the controls for each experiment and the statistical analysis was calculated.

### Example 17. Measurement of DPPH Radical Scavenging Activity of DDS

The radical scavenging activity was measured as the reducing power of DPPH included in methanol. DDS effect on the radical scavenging was measured based on the method that was previously researched [12] and Trolox was used as a positive control. The method for performing the above process may be simply described as follows: various concentrations (0.1-100 uM) of DDS dissolved in 0.5 ml of ethanol and Trolox were added to test tubes with 2.5 ml of methanol containing 200 uM of DPPH and then mixed. DPPH is a stable free radical, has a thick purple color, and its absorbance is at 517 nm. However, if it reacts with an antioxidant, it has a pale yellow color. The reaction mixture was maintained for 30 minutes at a room temperature without light and then the absorbance was measured at 517 nm. The free radical scavenging activity was calculated as the suppression rate by using the following equation: % suppression = [(Absorbance of Control - Absorbance of Sample)/Control Absorbance] x 100 .

### Example 18. Measurement of NADPH Oxidative Enzyme 4(NOX4) mRNA Level by using RT-PCT

RNA of the human diploid fibroblast was isolated by using Trizol Reagent (MRC Cincinnati, USA), and NOX4 and GAPDH were measured through RT-PCR by using Biometra T Gradient PCR (Biometra, Goettingen, Germany) machine. A specific NOX primer; 5'-GGTCCTTTTGGAAGTCCATTTGAGG-3' (Forward Primer) and 5'-CACAGCTGATTGATTCCGCTGAG-3' (Reverse Primer) ; Primer for GAPDH 5'-ACCACAGTCCATGCCATCAC-3' (Forward Primer) and 5'-TCCACCACCCTGTTGCTGTA-3' (Reverse Primer) were used as primers. DNA that was manipulated by PCR was isolated through 1.2 % agarose gel electrophoresis, stained with EtBr to be visualized and then taken a photography that can be shown through UV.

### Example 19. Measurement of Superoxide Anion in Cell (Cytoplasm)

The human diploid fibroblast was cultured at 96-well plate. The human diploid fibroblase was pretreated with DDS, DPI (30 min) and NAC, was treated with 1 mM of PQ for 30 minutes, and then cultured with 5µM of dihydroethidium (DHE)(a fluorescence material) without light at 37 for 15 minutes. The cell was twice washed with DPBS and Ethidium-DNA that fluoresces was immediately measured at excitation wavelength 515 nm and emission wavelength 590 nm by using Cary Eclipse fluorescence spectrophotometer (Varian, California, USA) machine.

### Example 20. Measurement of Superoxide Anion in Mitochondria

Mitochondria superoxide anion was measured by using the fluorescence material, i.e., MitoSOX Red. The fluorescence material that is reduced does not fluoresces before entering into a cell that breathe and the fluorescent probes that is oxidized in the cell selectively stain the mitochondria [13]. The human diploid fibroblase was pretreated with DDS, DPI (30 min) and NAC for 3 hours, was treated with 1 mM of PQ for 30 minutes, and then cultured with 5µM of MitoSOX Red that is a fluorescence material without light at 37 for 15 minutes. The cell was twice washed with DPBS and the fluorescence was immediately measured at excitation wavelength 515 nm and emission wavelength 590 nm by using CEFS [Cary Eclipse fluorescence spectrophotometer (Varian, California, USA)] machine.

### Example 21. Measurement of the Amounts of Mitochondria Complex and PKC Activated through Western Blot

The lysate of the human diploid fibroblast that was treated with DDS and PQ was heated by using a reagent buffer solution (50 mM Tris-HCl (pH 6.8), 2 % SDS, 0.14 M 2-mercaptoethanol, 10 % glycerol and 0.001 % bromophenol blue) and then isolated by an electrophoresis. The isolated sample was transferred to a nitrocellulose and analyzed through Western Blot using MS601 (Total OXPHOS Complexes Detection Kit, MitoSciences, containing antibodies against SDH30 subunit of complex II (5 µg/ml), Core2 subunit of complex III (0.2 µg/ml), COXII subunit of complex IV (2 µg/ml), and subunit α of F1-ATPase of complex V (0.2 µg/ml)), PCKpan-p, PCKα/β-p, PKCδ-p (Phospho-PKC antibody Sampler Kit, Cell Signaling Technology), PKCα, PCKβ, PKCδ (Life Technologies), β-action (Sigma), and the like. The measurement was performed by using a horseradish peroxygenase-conjugated secondary antibodies (Zymed) and ECL (Pierce). The results were shown through visualizing by using Autoradiographic film (Image Reader, LAS-3000Fujifilm, Japan).

### Example 22. Measurement of Calcium Amount in a Cell

The amount of Calcium in a cell was measured by using Fluo-4 (5 uM) that is a fluorescent material for labeling calcium and is well passed through a cell. The cell was cultured at 37 °C for 30 minutes and then maintained at a room temperature for 30 minutes without light in order for a complete de-esterification of AM ester in a cell. The fluorescent material that was left was removed except a cell by using HBSS containing 20 mM of HEPES (pH 7.4), and 100 ul of HBSS was added into wells containing cells, respectively. The fluorescent signal was measured at 494 nm for the excitation wavelength and 516 nm for the emission wavelength by using the multi-well fluorescence plate Eclipse fluorescence spectrophotometer (Varian, California, USA) [14]. The result was shown as Relative fluorescence units (RFUs).

### Example 23. Measurement of Membrane Potential of Mitochondria by Using DiOC6 Assay

The change of membrane potential (ΔΨm) of mitochondria was measured by using a cationic dye, DiOC6 that is a specific fluorescent material for mitochondria. An aspect of the change was measured by using Fluorescence Spectrophotometer [15, 16]. The cell was cultured in 40 nM DiOC6 for 15 minutes and then the fluorescent material that was left was removed by using PBS. The analysis was performed by using Cary Eclipse fluorescence spectrophotometer (Varian, California, USA), and then the excitation wavelength was measured at 480 nm and the emission wavelength was measured at 520 nm.

### Example 24. Staining of Mitochondria Structure by using MitoTracker Red (Imunofluorescence)

The human diploid fibroblasts were cultured at 24-well plate. After the above experiment materials, i.e., the human diploid fibroblasts, were pretreated and then were treated with PQ for 24 hours, the above cells were treated with MitoTracker Red CMXRos (50 nM), which is a staining material for mitochondria, at 37 °C for 30 minutes. The dye is a marker of function for marking mitochondria through marking a transmembrane potential of mitochondria in a cell. After culturing, the cells were washed with PBS and then fixed with 4 % of paraformaldehyde at 4 °C for overnight. Next day, the cells were treated with Blocking solution (1 % bovine serum albumin in PBS) at a room temperature for 1 hour. In addition, the cells were treated with DAPI at a room temperature for 10 minutes. After washing, the cells were covered with a cover glass and visualized through a fluorescence microscope.

### Example 25. Statistical Analysis

For the comparison of experiment results, the statistical significances as the differences between the groups were evaluated by using One-way ANOVA. It is admitted that the statistical significance is when the probability of significance is P<0.05 and P<0.005.

### <Results of Examples>

**1. Effect of Dapsone on Lifespan of Object**

**[Table 1]**

| Statistical Analysis I of experiment for extending lifespan | | | | | |
|---|---|---|---|---|---|
| **Figure** | **Strain** | **Feeding** | **Mean lifespan (Mean±SE)** | **p-Value** | **n** |
| **S2** | N2 | *folP* | 15.85±0.3 | | 109 |
| | N2 | *folP*+DDS | 17.43±0.4 | *p*<0.001 | 129 |
| (trial 2) | N2 | *folP* | 15.38±0.3 | | 96 |
| | N2 | *folP*+DDS | 17.87±0.4 | *p*<0.01 | 101 |
| (trial 3) | N2 | *folP* | 16.24±0.2 | | 161 |
| | N2 | *folP*+DDS | 16.90±0.3 | *p*=0.1355 | 128 |
| **S5** | N2 | HT115(L4440) | 20.29±0.5 | | 106 |
| | N2 | HT115(*pyk*-2 RNAi) | 20.66±0.6 | *p*=0.3479 | 116 |
| | *pyk-1(ok1754)* | HT115(L4440) | 23.57+0.5 | *p*<0.0001* | 133 |
| | *pyk-1(ok1754)* | HT115(*pyk-2* RNAi) | 23.71±0.4 | *P*=0.5637 | 135 |
| (trial 2) | N2 | HT115(L4440) | 19.20±0.5 | | 110 |
| | N2 | HT115(*pyk-2* RNAi) | 19.00±0.5 | *p*=0.9929 | 86 |
| | *pyk-1(ok1754)* | HT115(L4440) | 24.27±0.4 | *p*<0.0001* | 128 |
| | *pyk-1(ok1754)* | HT115(*pyk-2* RNAi) | 26.11±0.4 | *p*=0.0017 | 123 |
| (trial 3) | N2 | HT115(L4440) | 18.18±0.3 | | 99 |
| | N2 | HT115(*pyk-2* RNAi) | 19.00±0.3 | *p*=0.04 | 95 |
| | *pyk-1(ok1754)* | HT115(L4440) | 20.54±0.3 | *p*<0.0001* | 125 |
| | *pyk-1(ok1754)* | HT115(*pyk-2* RNAi) | 19.19±0.2 | *p*<0.0001 | 121 |
| **S7** | N2 | OP50+PABA | 18.04±0.4 | | 73 |
| | N2 | OP50+0.5mM DDS | 22.03±0.6 | *p*<0.0001** | 88 |
| | N2 | OP50+1mM DDS | 22.20±0.6 | *p*<0.0001** | 96 |

| | | | | | |
|---|---|---|---|---|---|
| SE means standard error of the mean. The total number of dying animals is represented by an n. *p*-values compare the experimental group with the control group of the upper row, except for *p*-value* and **, which are for the comparison with the N2 control worms. Additional experimental repeats are listed in the parentheses. | | | | | |

**[Table 2]**

| Statistical Analysis II of experiment for extending lifespan | | | | | |
|---|---|---|---|---|---|
| **Figure** | **Strain** | **Feeding *E.coli*** | **Mean lifespan (Mean±SE)** | ***p*-Value** | **n** |
| **1A** | N2 | OP50+PABA | 16.69±0.5 | | 100 |
| | N2 | OP50+PABA+DDS | 23.07±0.5 | *p*<0.0001 | 129 |
| (trial 2) | N2 | OP50+PABA | 21.18±0.4 | | 77 |
| | N2 | OP50+PABA+DDS | 24.31±0.6 | *p*<0.0001 | 100 |
| **1B** | N2 | OP50+PABA | 16.12±0.4 | | 106 |
| | N2 | OP50+PABA+DDS | 23.04±0.7 | *p*<0.0001 | 107 |
| (trial 2) | N2 | OP50+PABA | 22.92±0.7 | | 112 |
| | N2 | OP50+PABA+DDS | 34.26±0.8 | *p*<0.0001 | 128 |
| (trial 3) | N2 | OP50+PABA | 16.52±0.4 | | 93 |
| | N2 | OP50+PABA+DDS | 23.76±0.6 | *p*<0.0001 | 101 |
| (trial 4) | N2 | OP50+PABA | 14.24±0.3 | | 101 |
| | N2 | OP50+PABA+DDS | 18.87±0.4 | *p*<0.0001 | 106 |
| **2A** | N2 | OP50+PABA | 16.69±0.5 | | 100 |
| | *daf-16(cf1038)* | OP50+PABA | 11.62±0.3 | *p*<0.0001 | 84 |
| | *daf-16(cf1038)* | OP50+PABA+DDS | 15.69±0.5 | *p*=0.1291* | 78 |
| (trial 2) | N2 | OP50+PABA | 18.04±0.4 | | 73 |
| | *daf-16(cf1038)* | OP50+PABA | 15.29±0.3 | *p*<0.0001 | 98 |
| | *daf-16(cf1038)* | OP50+PABA+DDS | 17.40±0.3 | *p*=0.4234* | 100 |
| **3F** | N2 | OP50+PABA | 14.02±0.5 | | 95 |
| | N2 | OP50+PABA+DDS | 23.72±0.7 | *p*<0.0001 | 110 |
| | *pyk-1(ok1754)* | OP50+PABA | 17.66±0.6 | *p*<0.0001^{#} | 90 |
| | *pyk-1(ok1754)* | OP50+PABA+DDS | 21.92±0.8 | *p*<0.0001 | 101 |
| (trial 2) | N2 | OP50+PABA | 15.50±0.3 | | 88 |
| | N2 | OP50+PABA+DDS | 19.74±0.6 | *p*<0.0001 | 82 |
| | *pyk-1(ok1754)* | OP50+PABA | 17.73±0.5 | *p*<0.0001^{#} | 123 |
| | *pyk-1(ok1754)* | OP50+PABA+DDS | 23.37±0.6 | *p*<0.0001 125 | |

| | | | | | |
|---|---|---|---|---|---|
| SE means standard error of the mean. The use of 'n' represents the total number of animals examined. *p*-values compare the experimental group with the control group of the upper row except for *p*-value* and ^{#}, which are for the comparison with control N2 worms. Additional experimental repeats are listed in the parentheses. | | | | | |

*C. elegans* was used in order to research aging process and lifespan. After analyzing the lifespans of *C. elegans* that fed on *E. coli* treated with dapsone, the results were shown in Fig. 1, Fig. 2, Fig. 3, Table 1 and Table 2. As shown in Fig. 1, Fig. 2, Fig. 3, Table 1 and Table 2, it is shown that the lifespan of *C. elegans* that was treated with dapsone was significantly increased. At this time, since dapsone (DDS) is not well-dissolved in a water, a nutrient growth medium (NGM) agar plates, which is a culture medium of Escherichia coli OP50 that is used as a food of *C. elegans*, was treated with dapsone so that *C. elegans* could feed on dapsone while it grows. Moreover, since DDS competitively suppresses a para-aminobenzoic acid (PABA) thereby suppressing the synthesis of folic acid, *C. elegans* was cultured by treating PABA (10 uM, maintaining 80 % of growth rate of control, Fig. 1) thereby reducing the antibiotic effect of DDS. The treatment of PABA helps to growth bacteria, thereby suppressing the effect of dietary restriction due to the starvation of nematode. It can be shown that about 5 mg of DDS per a kg is accumulated by treating with 2 mM of DDS (Fig. 2). The dosage is similar to the one that is administrated for the patient with Hansen's disease [42]. The lifespan of *C. elegans* that were treated with PABA and DDS together was shown to be significantly long as compared with the control (Fig. 3, Table 1). The average lifespan and maximum lifespan of all *C. elegans* that were treated with DDS after L4-stage, which is adolescence of human, and during the whole life, were significantly increased as compared with the control (Fig. 3A, B). Moreover, as shown in the state at 23 days after adult, *C*. *elegans* that was treated with DDS had a more strong active movement (Fig. 4). The accumulation degree of lipofuscine (55) was measured for identifying that those results allow aging start delay or aging process (degree) extend. In *C*. *elegans* that fed on DDS during the whole life and after the adolescence, the generation of lipofuscin was delayed for several days (Fig. 5). From those results, it can be predicted that DDS allows aging start delay as compared with the control.

### 2. Mechanism for Extending Lifespan by Dapsone

Meanwhile, it is reported that the extension of lifespan of *C*. *elegans* is affected by various key factors; a reduced insulin signal, a dietary restriction, a reduced mitochondria function, and the like that none of key factors overlap [56]. Firstly, the experiment was performed by using daf-16 mutant (43) that is a key modulator of insulin signal as shown in Fig. 6 and Table 1 in order to identify the effects of insulin signal on the extension of lifespan among the possible mechanisms of dapsone (DDS). These results were shown that the lifespan of daf-16 mutant *C*. *elegans* that has a short lifespan were extended by DDS so that it can be known that DDS is not depended on the insulin signal for extending lifespan. As mentioned above, we could exclude the suppression of growth of bacteria, which was caused by DDS, by treating with DDS and PABA altogether in order to avoid the dietary restriction caused by DDS (Fig. 1). Therefore, we found out that the dietary restriction is not a reason for extending lifespan of *C. elegans* by treating with DDS. With the above results, it could be known that for identifying the growth of *C. elegans* that fed on the bacteria (57) lacking *folP* that is a direct target material of DDS, DDS was treated to the above *C. elegans* thereby living longer as compared with *C. elegans* as a control (Fig. 7, Table 2). Since the extension of lifespan of *C. elegans* by DDS is not depended to daf-16 and the dietary restriction, we next identified the effect of DDS on the mitochondria. We isolated the mitochondira from *C. elegans* and then identified the amount of mitochondrial complex V protein through Western Blot in order to identify the effects of DDS on the ATP production rate and function of mitochondria (Fig. 8). The amount of complex V was significantly decreased from the results of quantification analysis from *C. elegans* that was treated with DDS. In addition, ATP amount was decreased in *C. elegans* that was treated with DDS (Fig. 9). The oxygen uptake rate was meaningfully decreased in *C. elegans* that was treated with DDS (Fig. 10). As shown in these results, it can be known that DDS has an effect of suppressing the ROS generation. We measured H2O2 productions in *C. elegans* that was treated with PQ but not DDS, or was treated with PQ and DDS, in which PQ produces the active oxygen in a cell in order to identify DDS effect on suppressing ROS generation. We found that DDS meaningfully suppresses ROS generation by PQ (Fig. 11). Thus, it could be known that the survival rate of *C. elegans* that was treated with DDS in NGM liquid medium containing PQ (250 mM) was significantly increased as compared with *C*. *elegans* as a control (Fig. 12). From these results, it can be anticipated that DDS can increases the resistance of *C. elegans* about an oxidative stress. Interestingly, it could be shown that the amounts of mRAN in C. elegans NOX and ceDuox that are another source of ROS were significantly decreased by treating with DDS (Fig. 13). From these results, it can be anticipated that DDS affects to the cytoplasm as well as mitochondria. From this reason, it is very difficult to know how DDS can affect to the above reactions.

The mechanism of DDS as an antibiotic is well known. It is well known that DDS suppresses the growth of bacteria by competitively binding to PABA in an active place of DHPS enzyme that is required for synthesizing folic acid by the bacteria. However, since DHPS is not in eucaryocyte, the mechanism of function of DDS cannot be found in eucaryocyte. However, as shown in a cell experiment and the results in Caenorhabditis elegan, we were tried to identify the target protein of DDS in eucaryocyte in order to explain that DDS affects on the oxidative stress or has an effect as an antioxidant. The binding structure of DDS and DHPS is well known (58) and we analyzed the protein structure in order to find a candidate protein having a structure similar to a binding pocket of DHPS. We found out that a cytochrome P450 has a structure that is very well fitted on DDS. The cytochrome P450 is well known as a material that binds to a xenobiotics compound and decomposes and DDS is recognized and decomposed by P450 in *C. elegans*. Secondly, the pyruvate kinase is a candidate material (Fig. 14 and Fig. 15). Actually, the patient having a lack of PK has a hemolytic anemia and the above fact is a key side effect of Hansen's disease (60, 61). And, it is reported that the increased pyruvate content has relevance to the extension of lifespan (62). We were tried to find whether or not PK is a substantial target material of DDS for extending lifespan and DDS suppresses the biochemical activity of PK in vitro and in vivo (Fig. 16). In addition, as an unexpected result, *C*. *elegans* that fed on DDS has a high content of pyruvate as compared with a control (Fig. 17A). And, it is reported that the patient having Hansen's diseases that was administrated with DDS has a high content of pyruvate as compared with the control that was not administrated with DDS, like the above result (63). At this time, we concentrated on the gene of PK (pyruvate kinase) of *C. elegans*. The genome of *C. elegans* has *pyk-1* and *pyk-2* genes that are F25H5.3 and ZK593.1, i.e., two genes of PK, and *C. elegans* having *pyk-1* (ok1754) mutant that is a lack of *pyk-1* shows a high content of pyruvate (Fig. 17B). The inventors found out that the lifespan of *pyk-1* (ok1754) *C. elegans* that was not treated with DDS was extended as compared with N2 control worm but was a shorter than N2 that was treated with DDS (Fig. 18, Table 1). These results mean that there is a targeting by further treating with DDS rather than *pyk-1* on the extension of lifespan. Since there is not a test for using *pyk-1* mutant, we investigated *pyk-2* RNAi effect on lifespan. As shown in Fig. 19, *pyk-2* RNAi did not show any effect on lifespan for itself or even if it combines with *pyk-1* (ok1754) mutant. From the above results, it can be known that *pyk-2* does not have an effect of DDS. It is reported that *pyk-1* is mainly expressed in a muscle and *pyk-2* is mainly expressed in a small intestine in the previous researches (66), so that it can be known that the effect of DDS is composed of PK activity of muscle not a small intestine. From all the results, we can certainly know that the main target material of DDS of *C. elegans* is a muscle PK and there can be other unidentified target material. DDS target material can be identified through the search of protein that can react with DDS. Now, it is not easy to modulate the inhibition of PK activity by the mutant that can increase the pyruvate level, or DDS. As one possibility, there is a chance that further amino acid and pyruvate can be supplied through an autophagy. However, the gene relevant to the autophagy is not changed after treating with DDS in a transcription level, and LGG-1 that is a marker protein of the autophagy is not increased after treating with DDS, so that there is not a chance that further amino acid and pyruvate can be supplied through an autophagy (Fig. 21). As second possibility, DDS inhibits the reaction of other enzyme in TCA cycle thereby reducing the consumption of pyruvate. At the same time, the level of mitochondria complex V and the oxygen uptake rate were decreased by DDS. It can be known that the suppression of PK activity can suppress TCA cycle as a compensative mechanism. The above possibility can be explained through the results that isp-1 mutant *C*. *elegans* having a lack of mitochondria complex III and the extension of lifespan maintains a high level of pyruvate (Fig. 20). It can be concluded that the content of pyruvate is very important for extending lifespan from the above results.

Aging is caused by the complex process (reasons) of structural and biochemical changes in a single cell and complete object. The basic and accurate mechanism of aging is not well understood but it is clear that lifespan is relevant to the generation of active oxygen (64, 65). We found out that DDS allows the lifespan of *C*. *elegans* extend through the control of ROS generation from the research. Our results were shown that DDS is very important for extending lifespan of *C*. *elegans* and these results can be possible for human. Further researches should be performed for identifying that various other functions of DDS, i.e., immune response, metabolism, brain function, and the like affect lifespan.

Generally, patients take a dose as a standard concentration of 100 mg/day for a long time, but DDS with a low concentration could be more effective for extending lifespan because there is clinically a side effect within un-meaningful range (59). Thus, we found out that *C. elegans* that was treated with 1 mM and 0.5 mM of DDS of lower concentration show the effective effect for extending lifespan as much as the case of treating with 2 mM of DDS (Fig. 22, Table 2). The inventors found out that DDS that is used for treating a skin disease, malaria, and Hansen's disease can extend the lifespan of *C. elegans* and distributes the extension of lifespan by reducing ROS generation. Thus, it can be known that the administration of dapsone having low concentration can effectively extend the lifespan of human.

### II. Experiment of Modulating Aging in a Cell Level

### 1. DDS improves the cytotoxic that is induced by PQ.

In order to estimate the effect of DDS on the suppression of cytotoxic that is induced by PQ, the human diploid fibroblast was pretreated with various concentrations (0.1, 1, 5, 20 and 50 uM) of DDS, DPI (5 uM) and NAC (2 mM), and then was treated with 1 mM of PQ for 48 hours. DPI that is well known as a suppression material of NOX and DPI that is well known as an antioxidant were used as positive controls. The viability of cell was measured by using Cell Counting Kit (CCK-8). The viability of the human diploid fibroblast that was treated with PQ without any pretreatments was reduced to about 58.8 % as compared with the non-treated control. In contrast, the viability of the human diploid fibroblast that was pretreated with DDS was improved as compared with the cell having the cytotoxic of PQ. In addition, DPI and NAC that were used as positive controls show the effects of about 80 to 90 % suppression of cytotoxic that is induced by PQ as compared with the non-treated control (Fig. 23).

### 2. DDS suppresses the production of superoxide anion by PQ

The production of superoxide anion by PQ was measured. MitoTracker Red that is a fluorescent material used in order to observe the superoxide anion of mitochondria and dehydroethidium (DHE) as a superoxide anion of cytoplasm. The strong fluorescent material binds DNA when DHE is oxidized to oxoethidium by the superoxide anion [17]. It was identified through DHE assay that the superoxide anion was produced by PQ in the human diploid fibroblast and the produced superoxide anion was reduced depending upon the concentration of DDS. In addition, the production of superoxide anion was suppressed by DPI and NAC that were used as positive controls (Fig. 24A).

MitoTracker Red as a fluorescent material was used in order to measure the superoxide anion that is generated in mitochondria, in which the reduced fluorescent material does not fluoresce in the cell that actively breathe. However, when the mitochondria were oxidized by the superoxide anion, the fluorescent material enters in the cell and then fluoresces so that the superoxide anion can be measured [13]. The human diploid fibroblast that was treated with DDS suppresses the production of superoxide anion in the mitochondria depending upon the concentration of DDS (Fig. 24B) and also DPI and NAC that were used as positive controls suppress the production of superoxide anion in the mitochondria.

From these results, it can be known that DDS can suppress the superoxide anion in the mitochondria and the cytoplasm that were produced by PQ so that the cytotoxic that was induced by PQ can be controlled.

### 3. DDS does not improve the activity of removing ROS

In order to analyze the function of DDS as an antioxidant, the inventors firstly found out the effect of DDS on DPPH that is a free radical. However DDS does not show any improvement about the activity of removing ROS on DPPH in the test tube. Meanwhile, Trolox (water-soluble vitamin E) that was used as the control of antioxidant shows the strong activity of removing ROS (Fig. 25A).

Many antioxidants have an enzymatic oxidative protection activity relevant to the enzyme activity of removing ROS that is generated. Thus, the inventors evaluated the amounts of SOD1 and SOD2 that are the enzyme for removing ROS by treating with various concentrations of DDS into the human diploid fibroblast on the assumption that DDS can increase the amount and activity of enzyme for removing ROS. Interestingly, DDS ironically decreases the amounts of SOD1 and SOD2 (Fig. 25B).

From the above results, it can be known that DDS suppresses ROS production, not removes ROS that was produced. In order to research the antioxidant effect of DDS, the oxidative stress was induced by using paraquat that is well known as the material for producing the superoxide anion in the human diploid fibroblast.

### 4. DDS suppresses the NOX4 expression that is increased by PQ

NOX4 is a main isomer of NADPH oxidase gene in the fibroblast of human skin [18, 19] and the amount of ROS production is mainly determined by NOX4 expression. Thus, the suppression of NOX4 expression can cause the decrease of ROS production [20]. NOX4 and GAPDH were analyzed through RT-PCT in the human diploid fibroblast that was treated with 1 mM of PQ for 5 minutes. The amount of NOX4 was increased in the human diploid fibroblast that was treated with PQ. The human diploid fibroblasts were treated with various concentrations of DDS in order to analyzing whether or not DDS affects on the production of superoxide anion in the human diploid fibroblast that was treated with PQ. As shown in the above results, it can be shown that DDS decreases NOX4 expression. In addition, DPI can be used for identifying such that NOX4 is required for producing the superoxide anion by PQ in the human diploid fibroblast (Fig. 26).

### 5. DDS modulates the activity of PKC that is increased by PQ through the control of calcium.

It is reported that DDS can control the amount of calcium in a cell [21]. In order to identify the role of DDS on the calcium that is increased by PQ, the inventors measured the amounts of calcium under various conditions by using Fluo-4 that is a fluorescent material. DDS having the concentration that shows the effect of suppression on the oxidative reaction decreases the amount of calcium that is increased by PQ (Fig. 27).

It is reported that PKC isomer is relevant to the activity of NOX [22]. The conventional PKC (cPKC) or the isomers depending upon calcium (such as PKC α, β-I, β-II and γ) need calcium for their activities [23]. In order to analyze the type of NOX activity through the activity of PKC, the inventors investigated the effect of DDS on PKC, in which PQ induces a phosphorylation, through Western Blot. As a result, it could be shown that PQ increased PKCβ phosphorylation (activity) in the human diploid fibroblast and the increased phosphorylation was reduced by DDS (Fig. 28).

From these results, it could be known that PQ may increase PKC activity by increasing the amount of calcium and increase ROS production and DDS can suppress the above results by modulating the amount of calcium.

### 6. DDS modulates an insufficiency of mitochondria that is increased by PQ

An insufficiency of mitochondria is characterized by the production of superoxide anion, the damage of membrane potential, the damage of mitochondria DNA, the structural change, and the like.

In order to identify whether or not DDS is involved to the generation of the insufficiency of mitochondria, the inventors investigated the effect of DDS on the damage that is induced by PQ through various methods.

Firstly, the inventors evaluated the effect of DDS on the change of complex protein amount of mitochondria through Western Blot. DDS can recover all the amount of mitochondria complex that is reduced by PQ (Fig. 29A).

Secondly, the inventors investigated the change of membrane potential of mitochondria by using DiOC6 that is a fluorescent material. The membrane potential of mitochondria in the human diploid fibroblast that was treated with 1 mM of PQ for 24 hours was decreased to 76.8 % as compared with the control. For the cells that were pretreated with DDS (for 3 hours) and DPI (for 30 minutes), the decrease of membrane potential that was induced by PQ were meaningfully maintained (Fig. 29B).

Thirdly, the inventors evaluated the structural change of mitochondria by using MitoTracker fluorescent material. The mitochondria in the general human diploid fibroblast have a long flowing shape (Fig. 30, First part). The mitochondria can be well investigated in the region that shows MitoTracker positive (MT+) fluorescence in a cell and the cytoplasm can be investigated as MitoTracker negative (MT-) that has a light fluorescence [24]. The human diploid fibroblast was treated with 1 mM of PQ for 24 hours so that the dye such as a small spot shape that disappear MT+ region of mitochondria was shown (Fig. 30, Second part). For the mitochondria of the human diploid fibroblast that was pretreated with DDS and DPI and then was treated with PQ, the original shape was recovered, partially (Fig. 30, Third and Fourth parts).

### 7. Discussion

DDS is constantly being used for treating many skin diseases. In addition, DDS is recommended as the drug for treating Hansen's disease in WHO. Generally, DDS is used since 1940 and is taken a doge for the patients with Hansen's disease [25, 26]. It is reported that when the general patients take a DDS standard dosage (100 mg/day) for a long time, there is a very few or clinically not significant side effects [4]. When taking DDS for a long time, DDS can be distributed all the organs as the concentration similar to the concentration of blood plasma [27]. When the general patient with Hansan's disease takes 100 mg/day of DDS for 6 to 28 days, the concentration of blood plasma becomes about 1 to 20 uM. Moreover, DDS concentration that can suppress 50 % growth of human diploid fibroblast (IC50) is about 745 uM (the data is not shown). Thus, the inventors researched by using DDS concentration having stability. Although it is not clear whether or not DDS has an oxidative functions or anti-oxidative function, it is reported that DDS suppresses the superoxide anion in a neutrophil that is a kind of phagocyte in the recent thesis [21]. The recent results give meaning to our research such that the anti-oxidative effect of DDS was investigated in the human diploid fibroblast not the phagocyte. However, since ROS production is generally low in the human diploid fibroblast, paraquat (PQ) that is well known to specifically accelerate the production of superoxide anion was used for the research.

The cytotoxic through ROS production by PQ was mainly researched in the phagocyte [28]. The inventors found out that the cytotoxic by PQ was shown in the human diploid fibroblast not the phagocyte and DDS suppresses the above cytotoxic in the research (Fig. 23). The inventors proved that DDS has the effect of suppression on producing the superoxide anion in the mitochondria and cytoplasm that is induced by PQ (Fig. 24).

An antioxidant removes ROS that is produced, suppresses the production of new ROS, and recovers the oxidative damage [29]. The superoxide anion that is not removed may be changed into hydroxyl radical that has a more high activity that anion radicals and the hydroxyl radical may induce directly the damages on DNA, protein, lipid, and the like. Several antioxidants shows the anti-oxidative feature through the remove of superoxide anion radicals [30, 31], but the material for strongly suppressing the production of superoxide anion can also be used as an antioxidant.

According to the present invention, the inventors considered deeply that DDS may have the possibility for effectively suppressing the radical production even in the cell not the phygocyte as well as the phygocyte. In order to understand the effect of DDS, we investigated *in vitro* the activity for removing the radical on DPPH that is well known and then it could be known that DDS does not show the function for removing DPPH. Meanwhile, Trolox that was used as a positive control shows a strong effect for removing DPPH radical (Fig. 24A). In addition, the inventors investigated the amounts of SOD1 and SOD2 proteins that are an antioxidase for removing the superoxide anion that is produced. The inventors obtained the result such that DDS ironically reduces both amounts of proteins, such as SOD1 and SOD2 in the human diploid fibroblast (Fig. 25B). From these results, it can be known that DDS having an antioxidant function is not directly relevant to the radical removing system (that is, directly remove a radical or indirectly increase the enzyme for removing a radical). Thus, the inventors focused on the antioxidant function of DDS that suppresses the production of radical rather than removes the radical that is produced.

The oxidative stress that is induced by PQ is caused by the increase of NADPH oxidase (NOX) (but it is reported that the oxidative stress is not relevant to xanthine oxidase) [8] and the insufficiency of mitochondria [32, 33]. NOX uses oxygen molecular and NADPH as a substance and FAD as a co-enzyme. NOX enzyme is involved in modulating wide physiological functions, such as a cell survival, a cell differentiation, a cell proliferation, calcium signal, and calcium movement [20, 34]. NOX4 that is a key type of skin fibroblast produces ROS, in which the stimulation of TGFβ increases ROS production [35, 36]. In addition, the stimulation of angiotension increases the amount of NOX4 thereby increasing ROS in mesangial cells of kidney [37]. Those stimulations increase ROS production by modulating the transcriptional amount of NOX4. The inventors found out that PQ increases the amount of mRNA in the human diploid fibroblast and DDS suppresses the above function of PQ. Moreover, the inventors performed the experiment by using DPI that is well known material for specifically suppressing NOX so that it can be known that PQ produces ROS depending upon NOX4 in the human diploid fibroblast (Fig. 26).

There is a research that DDS is involved in modulating calcium [21]. However, there is no result that the amount of calcium is controlled by PQ in the human diploid fibroblast, but the inventors obtained the results that PQ increases calcium in a cell and the increased amount of calcium is suppressed by DDS (Fig. 27B). The increased calcium increases PKC activity that is depended upon calcium and the PKC is required for the activity of NOC. It is well known that the increase of superoxide anion by PQ is involved in PKC [7, 8]. Thus, the inventors were tried to find out whether or not DDS can modulate the activity of PKC. The inventors found out that PKC phosphorylation that was induced by PQ was inhibited in the human diploid fibroblast (Fig. 27B).

Based on the above results, it can be known that DDS recovers the amount of calcium in a cell in the human diploid fibroblast that was treated with PQ, and suppresses the production of superoxide anion by reducing NOX having an activity that can be modulated by PKC.

Since the mitochondria are another key factor for producing ROS, it can be considered that it is involved in the toxicity and oxidative stress that are induced by PQ. The insufficiency of mitochondria by PQ is characterized by the high production of superoxide anion, the damage of membrane potential, the damage of mitochondria DNA, the structural change, and the like. The inventors identified that DDS can recover the abnormal characterizes of mitochondria that are induced by PQ in the human diploid fibroblast. It is reported that DPI has the unpredicted function of suppressing mitochondria oxidase as well as suppressing NOX [34]. In addition, it is found out that DPI suppresses the toxicity of mitochondria that is induced by PQ according to the present invention. Thus, it can be known that DDS is an effective material for suppressing PQ toxicity in the human diploid fibroblast.

In short, the inventors showed the result that DDS suppresses the mitochondria toxicity and oxidative stress that are induced by PQ in the human diploid fibroblast according to the present invention. These results were obtained from the suppression of the mitochondria changed and NOX4 by modulating calcium and PKC activity.

More specifically, it is required that the effectiveness of DDS is established for preventing the abnormal condition that is involved in the general oxidative stress according to the DDS research. However, it can be suggested that DDS can be a new drug for alleviating or minimizing the oxidative stress by suppressing the production of new radical not removing the produced radical. Thus, the inventors anticipate that DDS is being used for treating the patient with Hansen's diseases for a long time so that DDS stability can be assured and can provide the evidence that DDS contributes for living for a long time without any diseases.

### <References>

1. Wolf, R.; Matz, H.; Orion, E.; Tuzun, B.; Tuzun, Y. Dapsone. Dermatol Online J 8:2; 2002.
2. Bradshaw, T. P.; McMillan, D. C.; Crouch, R. K.; Jollow, D. J. Formation of free radicals and protein mixed disulfides in rat red cells exposed to dapsone hydroxylamine. Free Radic Biol Med 22:1183-1193; 1997.
3. Reilly, T. P.; Woster, P. M.; Svensson, C. K. Methemoglobin Formation by Hydroxylamine Metabolites of Sulfamethoxazole and Dapsone: Implications for Differences in Adverse Drug Reactions. J Pharmacol Exp Ther 288:951-959; 1999.
4. Zone, J. J. Dermatitis herpetiformis. Current Problems in Dermatology 3:6-41; 1991.
5. Niwa, Y.; Sakane, T.; Miyachi, Y. Dissociation of the inhibitory effect of dapsone on the generation of oxygen intermediates-in comparison with that of colchicine and various scavengers. Biochem Pharmacol 33:2355-2360; 1984.
6. Anderson, R.; Theron, A. J.; Ras, G. J. Regulation by the antioxidants ascorbate, cysteine, and dapsone of the increased extracellular and intracellular generation of reactive oxidants by activated phagocytes from cigarette smokers. Am Rev Respir Dis 135:1027-1032; 1987.
7. Martins Chaves, M.; Prates Rodrigues, A. L.; Pereira dos Reis, A.; Gerzstein, N. C.; Nogueira-Machado, J. A. Correlation between NADPH oxidase and protein kinase C in the ROS production by human granulocytes related to age. Gerontology 48:354-359; 2002.
8. Miller, R. L.; Sun, G. Y.; Sun, A. Y. Cytotoxicity of paraquat in microglial cells: Involvement of PKCdelta- and ERK1/2-dependent NADPH oxidase. Brain Res 1167:129-139; 2007.
9. Cocheme, H. M.; Murphy, M. P. Complex I is the major site of mitochondrial superoxide production by paraquat. J Biol Chem 283:1786-1798; 2008.
10. Yang, W.; Tiffany-Castiglioni, E. The bipyridyl herbicide paraquat induces proteasome dysfunction in human neuroblastoma SH-SY5Y cells. J Toxicol Environ Health A 70:1849-1857; 2007.
11. Boelsterli, U. A.; Lim, P. L. Mitochondrial abnormalities--a link to idiosyncratic drug hepatotoxicity? Toxicol Appl Pharmacol 220:92-107; 2007.
12. Suda, T.; Suzuki, Y.; Matsui, T.; Inoue, T.; Niide, O.; Yoshimaru, T.; Suzuki, H.; Ra, C.; Ochiai, T. Dapsone suppresses human neutrophil superoxide production and elastase release in a calcium-dependent manner. Br J Dermatol 152:887-895 2005.
13. Mellors, A.; Tappel, A. L. The inhibition of mitochondrial peroxidation by ubiquinone and ubiquinol. J Biol Chem 241:4353-4356; 1966.
14. Esposti, M. D.; Hatzinisiriou, I.; McLennan, H.; Ralph, S. Bcl-2 and mitochondrial oxygen radicals. New approaches with reactive oxygen species-sensitive probes. J Biol Chem 274:29831-29837; 1999.
15. Minta, A.; Kao, J. P.; Tsien, R. Y. Fluorescent indicators for cytosolic calcium based on rhodamine and fluorescein chromophores. J Biol Chem 264:8171-8178; 1989.
16. Hishita,T.; Tada-Oikawa, S.; Tohyama, K.; Miura, Y.; Nishihara, T.; Tohyama, Y.; Yoshida, Y.; Uchiyama, T.; Kawanishi, S. Caspase-3 activation by lysosomal enzymes in cytochrome c-independent apoptosis in myelodysplastic syndrome-derived cell line P39. Cancer Res 61:2878-2884; 2001.
17. Quillet-Mary, A.; Jaffrezou, J. P.; Mansat, V.; Bordier, C.; Naval, J.; Laurent, G. Implication of mitochondrial hydrogen peroxide generation in ceramide-induced apoptosis. J Biol Chem 272:21388-21395; 1997.
18. Miyata, K.; Rahman, M.; Shokoji, T.; Nagai, Y.; Zhang, G. X.; Sun, G. P.; Kimura, S.; Yukimura, T.; Kiyomoto, H.; Kohno, M.; Abe, Y.; Nishiyama, A. Aldosterone stimulates reactive oxygen species production through activation of NADPH oxidase in rat mesangial cells. J Am Soc Nephrol 16:2906-2912; 2005.
19. Rossary, A.; Arab, K.; Steghens, J. P. Polyunsaturated fatty acids modulate NOX 4 anion superoxide production in human fibroblasts. Biochem J 406:77-83; 2007.
20. Park, H. S.; Jin, D. K.; Shin, S. M.; Jang, M. K.; Longo,N.; Park, J. W.; Bae, D. S.; Bae, Y. S. Impaired generation of reactive oxygen species in leprechaunism through downregulation of Nox4. Diabetes 54:3175-3181; 2005.
21. Lambeth, J. D. NOX enzymes and the biology of reactive oxygen. Nat Rev Immunol 4:181-189; 2004.
22. Inoguchi, T.; Sonta, T.; Tsubouchi, H.; Etoh, T.; Kakimoto, M.; Sonoda, N.; Sato, N.; Sekiguchi, N.; Kobayashi, K.; Sumimoto, H.; Utsumi, H.; Nawata, H. Protein kinase C-dependent increase in reactive oxygen species (ROS) production in vascular tissues of diabetes: role of vascular NAD(P)H oxidase. J Am Soc Nephrol 14:S227-232; 2003.
23. Coppi, A. A.; Lesniak, J.; Zieba, D.; Schanne, F. A. The effects of lead on PKC isoforms. Ann N Y Acad Sci 919:304-306; 2000.
24. Matylevitch, N. P.; Schuschereba, S. T.; Mata, J. R.; Gilligan, G. R.; Lawlor, D. F.; Goodwin, C. W.; Bowman, P. D. Apoptosis and accidental cell death in cultured human keratinocytes after thermal injury. Am J Pathol 153:567-577: 1998.
25. Doull, J. A. SULFONE THERAPY OF LEPROSY. BACKGROUND, EARLY HISTORY AND PRESENT STATUS. Int J Lepr 31:143-160; 1963.
26. Wozel, G. The story of sulfones in tropical medicine and dermatology. Int J Dermatol 28:17-21; 1989.
27. Zuidema, J.; Hilbers-Modderman, E. S.; Merkus, F. W. Clinical pharmacokinetics of dapsone. Clin Pharmacokinet 11:299-315; 1986.
28. Fukushima, T.; Tanaka, K.; LIM, H. J.; Moriyama, M. Mechanism of Cytotoxicity of Paraquat. Environmental Health and Preventive Medicine 7:891-894; 2002 No. 3 p.89
29. Halliwell, B. Reactive oxygen species in living systems: source, biochemistry, and role in human disease. Am J Med 91:14S-22S; 1991.
30. Robak, J.; Gryglewski, R. J. Flavonoids are scavengers of superoxide anions. Biochem Pharmacol 37:837-841; 1988.
31. Sanz, M. J.; Ferrandiz, M. L.; Cejudo, M.; Terencio, M. C.; Gil, B.; Bustos, G.; Ubeda, A.; Gunasegaran, R.; Alcaraz, M. J. Influence of a series of natural flavonoids on free radical generating systems and oxidative stress. Xenobiotica 24:689-699; 1994.
32. Thakar, J. H.; Hassan, M. N. Effects of 1-methyl-4-phenyl-1,2,3,6-tetrahydropyridine (MPTP), cyperquat (MPP+) and paraquat on isolated mitochondria from rat striatum, cortex and liver. Life Sci 43:143-149; 1988.
33. Tomita, M. Comparison of one-electron reduction activity against the bipyridylium herbicides, paraquat and diquat, in microsomal and mitochondrial fractions of liver, lung and kidney (in vitro). Biochem Pharmacol 42:303-309; 1991.
34. Bedard, K.; Krause, K. H. The NOX family of ROS-generating NADPH oxidases: physiology and pathophysiology. Physiol Rev 87:245-313; 2007.
35. Cucoranu, I.; Clempus, R.; Dikalova, A.; Phelan, P. J.; Ariyan, S.; Dikalov, S.; Sorescu, D. NAD(P)H oxidase 4 mediates transforming growth factor-betal -induced differentiation of cardiac fibroblasts into myofibroblasts. Circ Res 97:900-907; 2005.
36. Sturrock, A.; Cahill, B.; Norman, K.; Huecksteadt, T. P.; Hill, K.; Sanders, K.; Karwande, S. V.; Stringham, J. C.; Bull, D. A.; Gleich, M.; Kennedy, T. P.; Hoidal, J. R. Transforming growth factor-betal induces Nox4 NAD(P)H oxidase and reactive oxygen species-dependent proliferation in human pulmonary artery smooth muscle cells. Am J Physiol Lung Cell Mol Physiol 290:L661-L673; 2006.
37. Gorin, Y.; Ricono, J. M.; Kim, N. H.; Bhandari, B.; Choudhury, G. G.; Abboud, H. E. Nox4 mediates angiotensin II-induced activation of Akt/protein kinase B in mesangial cells. Am J Physiol Renal Physiol 285:F219-229; 2003.
38. L. I. Grad, L. C. Sayles, B. D. Lemire, Proc Natl Acad Sci U S A 102: 2005.
39. V. Chavez, A. Mohri-Shiomi, A. Maadani, L. A. Vega, D. A. Garsin, Genetics 176:1567 ;2007.
40. Wozel, G. The story of sulfones in tropical medicine and dermatology. Int J Dermatol :17-21; 1989.
41. Campisi J., Cell, 84:497-500; 1996.
42. Zuidema J, Hilbers-Modderman E, Merkus F. Clinical pharmacokinetics of dapsone. Clin Pharmacokinet 1986; 11:299-315.
43. Ogg, S., S. Paradis, et al. "The Fork head transcription factor DAF-16 transduces insulin-like metabolic and longevity signals in C. elegans" Nature389(6654): 994-9: 1997.
44. Dillin, A., A. L. Hsu, et al. "Rates of behavior and aging specified by mitochondrial function during development." Science298(5602): 2398-401 : 2002.
45. Feng, J., F. Bussiere, et al. "Mitochondrial electron transport is a key determinant of life span in C. elegans" Dev Cell1 (5): 633-44:2001.
46. Edens, W. A., Sharling, L., Cheng, G., Shapira, R., Kinkade, J. M., and Lee, T. J. Cell. Biol. 154: 879-891:2001.
47. R. Woolson, in Statistical Methods for the Analysis of Biomedical Data (Wiley, New York, 1987).
48. S. Kwadijk, J. S. Torano, Biomed Chromatogr 16, 203 (May, 2002).
49. M. Keaney, F. Matthijssens, M. Sharpe, J. Vanfleteren, D. Gems, Free Radic Biol Med 37, 239 (Jul 15, 2004).
50. B. P. Braeckman, K. Houthoofd, A. De Vreese, J. R. Vanfleteren, Mech Ageing Dev 123, 105 (Jan, 2002).
51. S. S. Lee et al., Nat Genet 33, 40 (Jan, 2003).
52. L. Holm, S. Kaariainen, P. Rosenstrom, A. Schenkel, Bioinformatics 24, 2780 (Dec 1, 2008).
53. P. Emsley, K. Cowtan, Acta Crystallogr D Biol Crystallogr 60, 2126 (Dec, 2004).
54. H. U. Bergmeyer, K. Gawehn, M. Grassl, in Methods of Enzymatic Analysis. (Academic Press, New York, 1974), vol. 1, pp. 509-10.
55. M. R. Klass, Mech Ageing Dev 6, 413 (Nov-Dec, 1977).
56. S. Wolff, A. Dillin, Exp Gerontol 41, 894 (Oct, 2006).
57. G. Vedantam, G. G. Guay, N. E. Austria, S. Z. Doktor, B. P. Nichols, Antimicrob Agents Chemother 42, 88 (Jan, 1998).
58. L. Holm, S. Kaariainen, P. Rosenstrom, A. Schenkel, Bioinformatics 24, 2780 (Dec 1, 2008).
59. J. J. Zone, Curr Prob Dermatol 3, 3 (1991).
60. S. J. Grossman, D. J. Jollow, J Pharmacol Exp Ther 244, 118 (Jan, 1988).
61. A. S. Keitt, Am J Med 41, 762 (Nov, 1966).
62. J. H. Park et al., Neurosci Lett 413, 265 (Feb 21, 2007).
63. S. N. Sinha, S. C. Gupta, A. K. Bajaj, N. P. Srivastava, T. N. Mehrotra, Int J Lepr Other Mycobact Dis 50, 468 (Dec, 1982).
64. D. Harman, J Gerontol 11, 298 (Jul, 1956).
65. A. Sanz, R. Pamplona, G. Barja, Antioxid Redox Signal 8, 582 (Mar-Apr, 2006).
66. R. Hunt-Newbury et al., PLoS Biol 5, e237 (Sep, 2007).

## Claims

1. A composition containing dapsone as an effective component for controlling aging and extending lifespan.

2. The composition of claim 1, wherein the composition maintains a low energy level of mitochondria.

3. The composition of claim 1, wherein the composition reduces the level of Duox.

4. A food or drink prepared by using the composition according to claim 1 as a raw material for alleviating aging.

5. A cosmetic composition prepared by using the composition according to claim 1 as a raw material for alleviating aging of skin cell.

6. A pharmaceutical composition prepared by using the composition according to claim 1 as a raw material for alleviating aging of object or recovering the biological function of aging cell.

7. A method for controlling aging of cell, comprising treating with the composition according to claim 1 to a cell.

8. A method for applying the composition according to claim 1 to an object for preventing or alleviating the symptoms or diseases that is caused by cell aging.

9. A method for extending lifespan of object, comprising applying the effective amount of dapsone to an object.

10. The method of claim 9, wherein the object is an animal.

11. The method of claim 9, wherein the animal is human.

12. The method of claim 9, wherein the animal is *Caenorhabditis elegans*.


SEQUENCE LISTING

<110>    Seoul National University Industry Foundation

<120>    A Composition for Regulating Cellular Senescens and/or for
         Increasing Lifespan comprising Dapsone as Active Ingredient

<130>    dp-2008-0123

<150>    KR10-2008-72762
<151>    2008-07-25

<160>    28

<170>    KopatentIn 1.71

<210>    1
<211>    20
<212>    DNA
<213>    Artificial Sequence

<220>
<223>    ceDuox1-f

<400>    1
cttcacaccg ttggacattg                                                    20

<210>    2
<211>    20
<212>    DNA
<213>    Artificial Sequence

<220>
<223>    ceDuox1-r

<400>    2
gaagatgtgt gagccggaat                                                    20

<210>    3
<211>    20
<212>    DNA
<213>    Artificial Sequence

<220>
<223>    actin-f

<400>    3
tcgtaggact tctcgaggga                                                    20

<210>    4
<211>    20
<212>    DNA
<213>    Artificial Sequence

<220>
<223>    actin-r


<400>    4
atgttgccgc tctcgtagtt                                                    20


<210>    5
<211>    25
<212>    DNA
<213>    Artificial Sequence

<220>
<223>    NOX-f


<400>    5
ggtccttttg gaagtccatt tgagg                                              25


<210>    6
<211>    23
<212>    DNA
<213>    Artificial Sequence

<220>
<223>    NOX-r


<400>    6
cacagctgat tgattccgct gag                                                23


<210>    7
<211>    20
<212>    DNA
<213>    Artificial Sequence

<220>
<223>    GAPDH-f


<400>    7
accacagtcc atgccatcac                                                    20


<210>    8
<211>    20
<212>    DNA
<213>    Artificial Sequence

<220>
<223>    GAPDH-r


<400>    8
tccaccaccc tgttgctgta                                                    20


<210>    9

<211>    20
<212>    DNA
<213>    Artificial Sequence

<220>
<223>    eat2-f


<400>    9
gcaaattccc catggtacac                                                    20


<210>    10
<211>    20
<212>    DNA
<213>    Artificial Sequence

<220>
<223>    eat2-r


<400>    10
catggaaagt gagcacgaga                                                    20


<210>    11
<211>    20
<212>    DNA
<213>    Artificial Sequence

<220>
<223>    eat3-f


<400>    11
aggctgcatc agaacgtctt                                                    20


<210>    12
<211>    20
<212>    DNA
<213>    Artificial Sequence

<220>
<223>    eat3-r


<400>    12
tgtttgtgct ctggatctgc                                                    20


<210>    13
<211>    20
<212>    DNA
<213>    Artificial Sequence

<220>
<223>    bec1-f


<400>    13

caaagaaggc cagattcagc                                                    20

<210>  14
<211>  20
<212>  DNA
<213>  Artificial Sequence

<220>
<223>  bec1-r

<400>  14
cgttgtcgga tggttttctt                                                    20

<210>  15
<211>  20
<212>  DNA
<213>  Artificial Sequence

<220>
<223>  let-512 /VPS34-f

<400>  15
tatgcgagtc tccacgtcag                                                    20

<210>  16
<211>  20
<212>  DNA
<213>  Artificial Sequence

<220>
<223>  let-512 /VPS34-r

<400>  16
ccaatcgatc ctttgcttgt                                                    20

<210>  17
<211>  20
<212>  DNA
<213>  Artificial Sequence

<220>
<223>  T22H9.2 (atg9)-f

<400>  17
cacttcaacg agcttgacca                                                    20

<210>  18
<211>  20
<212>  DNA
<213>  Artificial Sequence

<220>

<223>    T22H9.2 (atg9)-r

<400>    18
gtgatgacgt gttccacctg                                                    20

<210>    19
<211>    20
<212>    DNA
<213>    Artificial Sequence

<220>
<223>    atgr-18 (atg-18)-f

<400>    19
caggaagcac tgacactgga                                                    20

<210>    20
<211>    20
<212>    DNA
<213>    Artificial Sequence

<220>
<223>    atgr-18 (atg-18)-r

<400>    20
aaagagccga tgtccatttg                                                    20

<210>    21
<211>    20
<212>    DNA
<213>    Artificial Sequence

<220>
<223>    lgg-3 (atg-12)-f

<400>    21
tgaaattgcg aaaactgctg                                                    20

<210>    22
<211>    20
<212>    DNA
<213>    Artificial Sequence

<220>
<223>    lgg-3 (atg-12)-r

<400>    22
gtaggccggt gtaatgctgt                                                    20

<210>    23
<211>    20

<212>    DNA
<213>    Artificial Sequence

<220>
<223>    atgr-5 (atg-5)-f


<400>    23
cgaatttgca cacattccac                                                    20


<210>    24
<211>    20
<212>    DNA
<213>    Artificial Sequence

<220>
<223>    atgr-5 (atg-5)-r


<400>    24
tccgttgagg atgatgatga                                                    20


<210>    25
<211>    20
<212>    DNA
<213>    Artificial Sequence

<220>
<223>    lamp1-f


<400>    25
caacgcttac aagtgctcca                                                    20


<210>    26
<211>    20
<212>    DNA
<213>    Artificial Sequence

<220>
<223>    lamp1-r


<400>    26
acgacgattg ggacaacttc                                                    20


<210>    27
<211>    20
<212>    DNA
<213>    Artificial Sequence

<220>
<223>    lamp2-f


<400>    27
ggccaaaaga aacttgtcca                                                    20

<210> 28
<211> 20
<212> DNA
<213> Artificial Sequence

<220>
<223> lamp2-r

<400> 28
tttcgtcatt ggaagctgtg 20